(19) Europäisches Patentamt
European Patent Office
Office européen des brevets

(11) **EP 3 628 678 A1**

(12) **EUROPEAN PATENT APPLICATION**

(43) Date of publication:
**01.04.2020 Bulletin 2020/14**

(51) Int Cl.:
*C07K 14/135* [(2006.01)]   *C12N 7/00* [(2006.01)]

(21) Application number: **18197259.7**

(22) Date of filing: **27.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ecole Polytechnique Federale De Lausanne (EPFL) EPFL-TTO 1015 Lausanne (CH)**

(72) Inventors:
• **CORREIA, Bruno**
  **1015 Lausanne (CH)**
• **SESTERHENN, Fabian**
  **1015 Lausanne (CH)**

(74) Representative: **Williams, Gareth Owen**
**Marks & Clerk LLP**
**62-68 Hills Road**
**Cambridge CB2 1LA (GB)**

(54) **IMMUNOGEN**

(57)    Polypeptides useful in the preparation of vaccine compositions against RSV are provided. Also disclosed are methods of enhancing subdominant antibody responses in a subject.

Fig. 4

**Description**

FIELD OF THE INVENTION

The present invention relates to a polypeptide, which may be used as an immunogen to provoke an immune response. The invention further relates to a fusion polypeptide comprising said polypeptide fused to a carrier polypeptide; and to a vaccine composition comprising the polypeptide or the fusion polypeptide. Aspects of the invention further relate to methods for enhancing a subdominant antibody response in a subject.

BACKGROUND OF THE INVENTION

Throughout the last decades, vaccination has been a key countermeasure to control and eradicate infectious diseases. However, many pathogens (Respiratory Syncytial Virus (RSV), Influenza, Dengue and others) evade the immune system mounting antibody responses that fail to confer broad and potent protection against reinfection, and, in some cases, mediating disease enhancement. Deep profiling of human B-cells often reveals potent neutralizing antibodies emerging from natural infection, but are generally subdominant. A major challenge for next-generation vaccines is to overcome established immunodominance hierarchies, and focus antibody responses on crucial neutralization epitopes.

Vaccination has proven to be one of the most successful medical interventions to reduce the burden of infectious diseases (*1*), and the major correlate of vaccine-induced immunity is induction of neutralizing antibodies that block infection (*2*). However, classical vaccine approaches relying on inactivated or attenuated pathogen formulations have failed to induce protective immunity against numerous important pathogens, urging the need for novel vaccine development strategies. Structure-based approaches for immunogen design have emerged as promising strategies to elicit antibody responses focused on structurally defined epitopes sensitive to antibody mediated neutralization (*3*).

In recent years, advances in high-throughput B-cell technologies have revealed an impressive wealth of potently neutralizing antibodies (nAbs) for different pathogens which have resisted the traditional means of vaccine development for several decades, including HIV-1 (*4*), Influenza (*5*), Respiratory Syncytial Virus (RSV) (*6, 7*), Zika (*8, 9*), Dengue (*10*) and others (*11-13*). Many of these antibodies have been structurally characterized in complex with their viral target proteins, unveiling the atomic details of neutralization-sensitive epitopes (*8, 14, 15*). The large-scale campaigns in antibody isolation, together with detailed functional and structural studies have provided comprehensive antigenic maps of the viral fusion proteins, which delineate epitopes susceptible to antibody-mediated neutralization and provide a roadmap for rational and structure-based vaccine design approaches.

The conceptual framework to leverage neutralizing antibody-defined epitopes for vaccine development is commonly referred to as reverse vaccinology (*16, 17*). Although the reverse vaccinology inspired approaches have yielded a number of exciting advances in the last decade, the design of immunogen that elicit such focused antibody responses remains challenging. Successful examples of structure-based immunogen design approaches include the conformational stabilization of RSVF in its prefusion state (*18*), yielding superior serum neutralization titers when compared to its postfusion conformation. In the case of Influenza, several epitopes targeted by broadly neutralizing antibodies (bnAbs) were identified within the hemagglutinin (HA) stem domain, and an HA stem-only immunogen elicited a broader neutralizing antibody response than that of full length HA (*19, 20*). Commonly, these approaches have aimed to focus antibody responses on specific conformations or subdomains of viral proteins. In a more aggressive approach, Correia et al. (*21*) computationally designed a synthetic immunogen presenting the RSV antigenic site II, and provided a proof-of-principle for the induction of RSV neutralizing activity mediated by a single epitope in non-human primates.

RSV, amongst other pathogens, is a striking example for the major need to be able to elicit epitope-focused antibody responses. While the causes of the failure of 1960's formalin inactivated RSV vaccine are not fully clear, several reports have suggested that the induction of antibodies targeting non-neutralizing and structurally altered neutralization epitopes may have played a role on the disease enhancement observed upon later infection (*22-24*). Despite more than 50 years of intensive research, a licensed vaccine has remained elusive, and RSV induced severe lower respiratory tract infections continue to cause more than three million hospitalizations annually. Thus far, the only prophylactic therapy for individuals at high risk for severe RSV disease it the passive immunization with Synagis®, a monoclonal antibody targeting antigenic site II of RSVF (*25*).

A major challenge associated with RSV, Influenza and other pathogens is posed by the absence of a potent and long-lasting immune response upon natural infection. While a single exposure to pathogens like Poliovirus confers life-long immunity, RSV, Influenza and other pathogens have developed mechanisms to evade antibody responses that are sufficiently potent to confer long-lasting protection, thereby allowing such pathogens to infect humans repeatedly throughout their life (*26*). One of the major factors hindering the induction of long-lasting protection after the first infection is related to the antibody specificities induced. Upon exposure to the pathogen the human-elicited antibody responses predominantly target non-neutralizing or strain-specific antigenic sites, whereas potent bnAbs are subdominant (*27*).

This phenomenon is generally referred to as B-cell immunodominance, which describes the unbalanced immunogenicity of certain antigenic sites within an antigen, favoring strain specific, variable, non-neutralizing epitopes to the detriment of conserved, neutralization sensitive epitopes (*28*). It is rather unclear which factors determine the antigenicity of specific epitopes, making the categorization of immunodominant and subdominant epitopes an empirical classification based on serological analysis. Importantly, the presence of high levels of antibodies directed against immunodominant epitopes can sterically mask surrounding subdominant epitopes that may be those targeted by bnAbs, preventing the immune system from mounting a productive antibody response against subdominant epitopes and potentially limiting vaccination efficacy (*27-30*).

The immunodominance hierarchy is established within the germinal center (GC), where B-cells undergo a binding affinity-based competition for the available antigen and subsequently initiate a clonal expansion stage, ultimately becoming long-lived plasma cells or memory B-cells (*31*). Controlling this competition and driving antibody responses towards the increased recognition of structurally defined, subdominant epitopes is of primary importance to enable the development of novel vaccines to fight pathogens which have resisted to traditional strategies. One of the few strategies to guide antibody maturation was tested in the HIV field and is referred to as germline targeting, which relies on the activation and expansion of rare, but specific B-cell lineages in naive individuals (*32, 33*). In contrast, under conditions of preexisting immunity acquired during natural infection or previous vaccination, the challenge is to manipulate already established B-cell immunodominance hierarchies, and reshape serum antibody responses towards desired specificities. In an indirect approach towards increasing subdominant B-cell populations, Silva et al. (*34*) have shown that the targeted suppression of immunodominant clones during an active GC reaction can allow subdominant B-cell populations to overtake the GC response. Alternative approaches used heterologous prime boost immunization regimens with either alternative viral strains or rationally modified versions of the priming immunogen (*35-38*) aiming to steer antibody responses towards more conserved domains. However, leveraging structural information of defined neutralization epitopes to steer bulk antibody responses towards specific, well characterized single epitopes remains an unmet challenge.

WO2006/117456 describes methods for preparation of soluble N-proteins from Paramyxoviridae, and in particular from RSV (RSV N-protein, RSVN). The publication further suggests use of said N-proteins as a vaccine composition. WO2007/119011 describes use of RSVN fusion proteins, with the RSVN being used as a carrier in order to present antigens to a cell, to provoke an immune response.

Correia et al (reference 21; 2014, Nature 507:201-206) show that such carriers can be used to present a computationally-designed polypeptide which mimics epitopes from RSV antigenic site II, and further that this can be used to induce RSV neutralising antibodies in primate test subjects with titres comparable to those induced by natural human infection. However, neutralising antibodies were not detected in test mice.

The present inventors have now shown that a modified computationally-designed polypeptide provides an improved immune response, including the generation of neutralising antibody responses in naive test mice. Further, the inventors have shown that the modified polypeptide can be used to enhance a subdominant antibody response in test subjects previously exposed to RSV antigens.

SUMMARY OF THE INVENTION

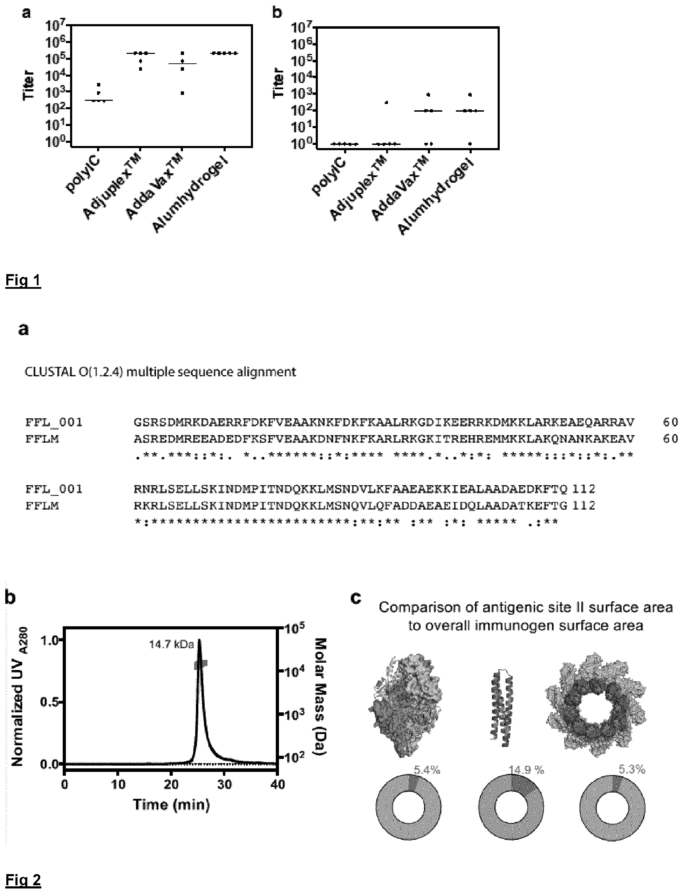

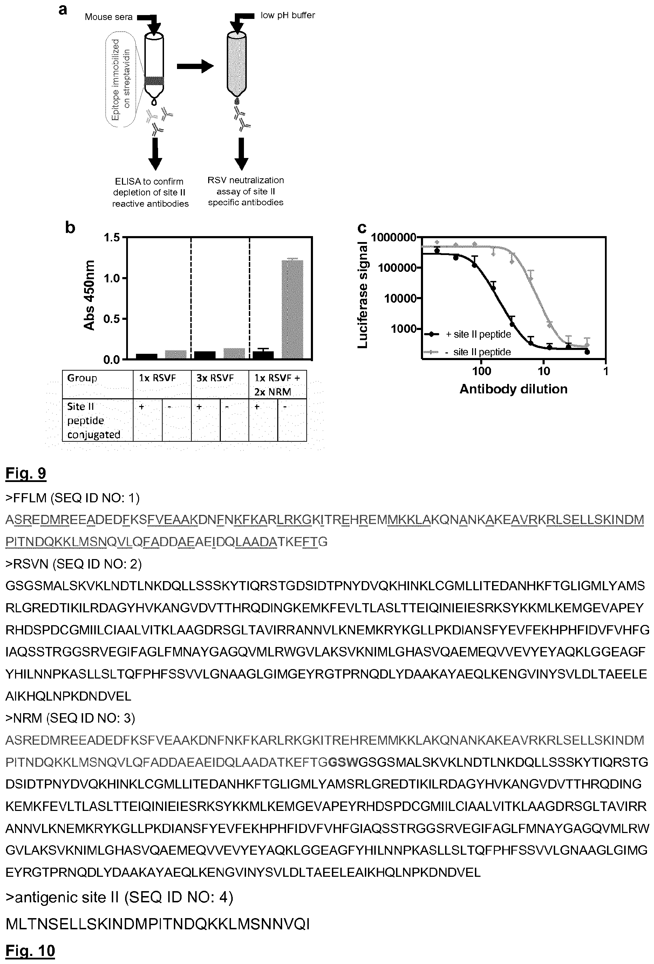

According to a first aspect of the present invention, there is provided a polypeptide comprising or consisting of an amino acid sequence according to SEQ ID NO: 1. ("an FFLM polypeptide"). Also provided is a polypeptide comprising an amino acid sequence which is 75%, 80%, 85%, 90%, 91%, 92%, 93%, 94%, 95%, 96%, 97%, 98% or 99% identical to that of SEQ ID NO: 1. The amino acid sequence may comprise one, two, three, four, five or more insertions, deletions, and/or substitutions, provided the resulting sequences result in immunogenicity when administered to a subject. Fig. 2a shows an alignment of FFLM and the FFL_001 sequence; identical residues are indicated with an asterisk (and are underlined in SEQ ID NO: 1 shown in Fig. 10). Where the amino acid sequence of the invention differs from that of SEQ ID NO: 1, preferably those residues which are identical in FFLM and FFL_001 are unchanged. Any differences are preferably found in residues 1, 4, 8, 9, 11, 12, 13, 15, 16, 23, 24, 26, 31, 36, 38, 39, 41, 43, 44, 50, 51, 52, 54, 55, 57, 58, 62, 87, 90, 93, 94, 97, 98, 100, 101, 107, 108, 109, or 112 of SEQ ID NO: 1; and more preferably in residues 13, 31, 36, 93, 97, 101, 107, or 112. Conservative subsitutions are preferred in any or all of residues 1, 4, 8, 9, 11, 12, 15, 16, 23, 24, 26, 38, 39, 41, 43, 44, 50, 51, 52, 54, 55, 57, 58, 62, 87, 90, 94, 98, 100, 108, or 109. In certain embodiments, non-conservative substitutions may be made in any or all of residues 13, 31, 36, 93, 97, 101, 107, or 112.

The polypeptide preferably further comprises a carrier polypeptide. In preferred embodiments, the carrier polypeptide comprises or consists of the amino acid sequence of SEQ ID NO: 2, and in more preferred embodiments, the full polypeptide comprises or consists of the amino acid sequence of SEQ ID NO: 3. In alternative embodiments, the carrier polypeptide comprises or consists of an amino acid sequence which is 75%, 80%, 85%, 90%, 91%, 92%, 93%, 94%, 95%, 96%, 97%, 98% or 99% identical to that of SEQ ID NO: 2. The carrier polypeptide may be an N-protein from Paramyxoviridae, and preferably an N-protein from RSV (RSVN). In preferred embodiments, the carrier polypeptide is RSVN as described in WO2006/117456, or in WO2007/119011.

The invention still further provides a nucleic acid sequence encoding any of the polypeptide sequences disclosed herein; most preferred are nucleic acid sequences encoding the amino acid sequence of SEQ ID NO: 1, or of SEQ ID NO: 2, or of SEQ ID NO: 3. Also provided are expression vectors comprising the nucleic acid sequences; and cells, preferably prokaryotic cells, comprising the expression vectors and/or the nucleic acid sequences as herein defined.

Aspects of the invention further provide a nanoparticle comprising one or more copies of a polypeptide sequence according to any preceding aspect of the invention; in particular, one or more copies of a polypeptide comprising an FFLM polypeptide and a carrier polypeptide, preferably an RSVN polypeptide, as herein defined. In preferred embodiments, the nanoparticle comprises one or more copies of a polypeptide sequence comprising or consisting of the amino acid sequence of SEQ ID NO: 3. Preferably the nanoparticle comprises 10 or more copies of the polypeptide.

The invention further provides a polypeptide sequence or a nanoparticle as herein defined for use in therapy, preferably for use as a vaccine, and most preferably a vaccine against RSV. Aspects of the invention also provide nucleic acid sequences as herein defined, for use in therapy, preferably for use as a nucleic acid vaccine, and most preferably a vaccine against RSV. Further provided is the use of a polypeptide sequence or a nanoparticle as herein defined in the manufacture of a medicament, preferably a vaccine, more preferably a vaccine against RSV. Also provided is a vaccine composition comprising a polypeptide sequence or a nanoparticle as herein defined. The vaccine composition may further comprise one or more pharmaceutically acceptable carriers, and/or adjuvants. The adjuvant may be AS04, AS03, alhydrogel, and so forth.

In preferred embodiments, the vaccine is for administration to subjects, preferably human subjects, who have been previously exposed to RSV.

The vaccine composition of the invention may be administered via any route including, but not limited to, oral, intramuscular, parenteral, subcutaneous, intranasal, buccal, pulmonary, rectal, or intravenous administration.

Vaccine compositions of the invention may further comprise one or more additional immunogenic components; in preferred embodiments, this may be an additional RSV-derived protein or glycoprotein, and most preferably the RSVF glycoprotein, or an RSVF protein precursor (for example, the core peptide sequence). An example RSVF protein sequence is given in the UniProt KB database as entry AOA110BF16 (AOA110BF16_HRSV).

In further embodiments of the invention, there is provided a kit of parts comprising a vaccine composition comprising a polypeptide sequence or a nanoparticle as herein defined; and a further vaccine composition comprising an RSV-derived protein or glycoprotein, and most preferably the RSVF glycoprotein, or an RSVF protein precursor (for example, the core peptide sequence). The kit of parts may further comprise instructions for administering the two vaccine compositions.

Also provided is a combination product comprising a vaccine composition comprising a polypeptide sequence or a nanoparticle as herein defined; and a further vaccine composition comprising an additional RSV-derived protein or glycoprotein, and most preferably the RSVF glycoprotein, or an RSVF protein precursor (for example, the core peptide sequence). The vaccine compositions may be provided as a single composition, although they are preferably provided as separate compositions. Administration may be sequentially or simultaneously; sequential administration is preferred.

A further aspect of the invention provides a method for immunising a subject, preferably a human subject, against RSV, the method comprising administering a vaccine composition comprising a polypeptide sequence or a nanoparticle as herein defined. The method may further comprise, prior to said administration step, administering a further vaccine composition comprising an RSV-derived protein or glycoprotein, and most preferably the RSVF glycoprotein, or an RSVF protein precursor (for example, the core peptide sequence). Alternatively, or in addition, the vaccine composition(s) may be administered to a subject who has previously been exposed to RSV infection.

Where multiple vaccine compositions are to be administered, the vaccine composition comprising an RSV-derived protein or glycoprotein (eg, RSVF) is referred to as the "priming" vaccination; the vaccine composition comprising a polypeptide sequence or a nanoparticle as herein defined is referred to as the "boost" vaccination, giving a prime:boost administration schedule. The method may comprise administering multiple boost vaccinations. The prime and first boost vaccinations may be administered according to any suitable schedule; for example, the two vaccinations may be administered one, two, three, four or more weeks apart. Where multiple boost vaccinations are administered, these too may be administered according to any suitable schedule; for example, the two vaccinations may be administered one, two, three, four or more weeks apart. Preferably two boost vaccinations are administered.

A further aspect of the invention provides a method for boosting subdominant immune responses to a selected pathogen, the method comprising administering a first priming vaccine comprising an immunogen obtained or obtainable from said selected pathogen, wherein the immunogen provides a desired subdominant immune response and a dominant immune response; and subsequently administering a second boost vaccine comprising a synthetic immunogen, said synthetic immunogen comprising a polypeptide sequence designed to mimic a desired epitope recognised by said subdominant immune response, said polypeptide sequence being fused to a carrier polypeptide sequence. The synthetic immunogen is a heterologous immunogen. The pathogen may be a virus, and in preferred embodiments may be, for example influenza virus, HCV, HCMV, zika, Dengue, HIV, or RSV. Preferably the synthetic immunogen mimics a conserved neutralisation sensitive epitope, which is shared across two or more strains of said pathogen. For example, the conserved neutralisation sensitive epitope may be a non-surface antigen. The method may further comprise administering a further boost vaccine. The carrier polypeptide sequence may be selected so as to form nanoparticles comprising multiple polypeptide sequences.

Further provided is a first priming vaccine comprising an immunogen obtained or obtainable from a selected pathogen, wherein the immunogen provides a desired subdominant immune response and a dominant immune response; and a second boost vaccine comprising a synthetic immunogen, said synthetic immunogen comprising a polypeptide sequence designed to mimic a desired epitope recognised by said subdominant immune response, said polypeptide sequence being fused to a carrier polypeptide sequence; for use in a method for boosting subdominant immune responses to said selected pathogen.

BRIEF SUMMARY OF THE FIGURES

These and other aspects of the invention will now be described in detail, and with reference to the following figures.

**Fig. 1: Adjuvant screen for FFL_001 immunogen. a)** Immunogenicity of FFL_001 formulated in different adjuvants. Serum titers were determined against FFL_001 at day 56 of the immunization protocol. FFL_001 emulsed in Alhydrogel showed highest overall immunogenicity. **b)** Prefusion RSVF cross-reactivity of FFL_001 immunized mice after three immunizations. 4/5 mice immunized with FFL_001 formulated in Alhydrogel showed serum cross-reactivity with prefusion RSVF.

**Fig. 2: Homology guided resurfacing of FFL_001.** a) Sequence alignment of FFL_001 and FFLM. b) Resurfaced variant FFLM is monomeric in solution as assessed by size-exclusion coupled to an on-line multi-angle-light scattering detector. Determined mass in solution is 14.7 kDa $\pm$ 3.5 %, which is close to the theoretical molecular weight of 14.4 kDa. c Relative surface area of RSVF antigenic site II in prefusion RSVF (PDBID 4JHW), FFL_001 and FFLM models. The Motavizumab epitope is highlighted in red, blue patches indicate sequence changes of FFLM compared to FFL_001, and piecharts show the fraction of antigenic site II surface area compared to overall immunogen surface area. Solvent accessible surface area (SASA) was computed in PyMol in presence and absence of Motavizumab bound to every site II repetition. % SASA of antigenic site II is nearly identical between RSVF and NRM, whereas FFLM monomer shows approximately 3-fold higher surface area of antigenic site II, due to its small size.

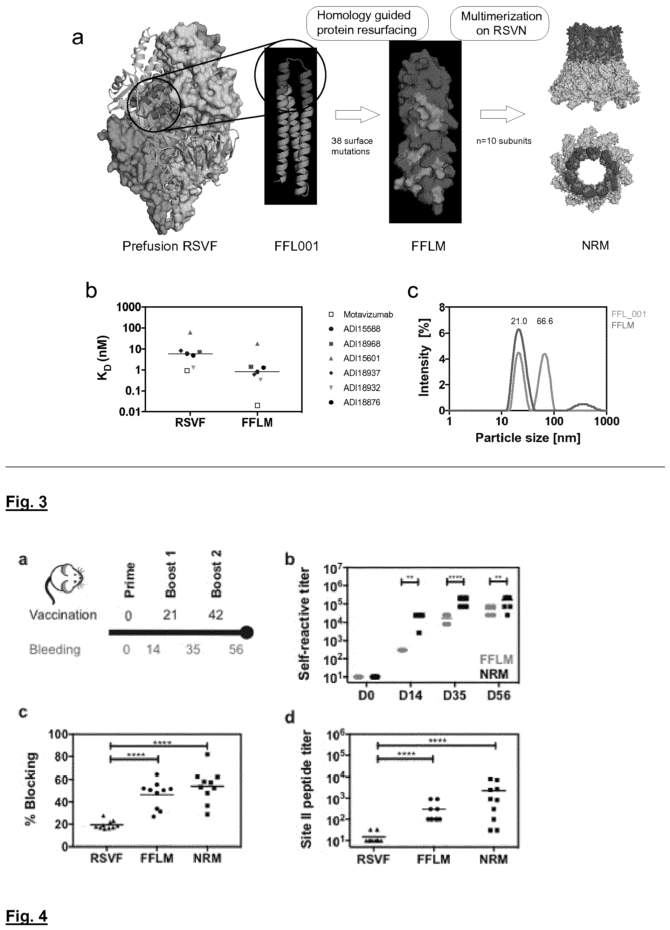

**Fig. 3. Design of an RSV based nanoparticle displaying a site II epitope-focused immunogen. a)** Structural model of prefusion RSVF trimer (PDBID: 4JHW), with two subunits shown as a grey surface and one subunit shown as lightblue cartoon representation with the epitope targeted by Motavizumab (antigenic site II) highlighted in red. FFL_001 was previously designed to present the site II epitope in a computationally designed scaffold. FFLM was designed by evolution guided resurfacing, and changes in amino acid identity are highlighted in blue. FFLM was genetically fused to the N-terminus of the RSV Nucleoprotein (RSVN), resulting in a high-density array of the epitope scaffold as shown by the structural model. **b)** Binding affinities of site II specific human neutralizing antibodies as measured by SPR. $K_D$s were measured with RSVF/FFLM as ligand and Fabs as analyte. **c)** Dynamic light scattering (DLS) profiles for FFL_001 and FFLM fused to RSVN. The FFL_001-RSVN fusion protein formed high-order oligomers in solution ($\approx$66.6 nm of median diameter), whereas the resurfaced FFLM-RSVN fusion protein (NRM) was monodisperse with a median diameter of 21 nm.

**Fig. 4. Immunogenicity and quantification of site II specific antibody responses. a)** Immunization scheme. Balb/c mice were immunized three times on days 0, 21 and 42, and blood was drawn 14 days after each vaccination. **b)** Serum antibody titers elicited by FFLM and NRM at different timepoints measured by ELISA against the respective immunogen. NRM shows significantly increased immunogenicity at day 14, 35 and 56 (** p = 0.008, *** p < 0.0001, Mann-Whitney U test). **c)** SPR competition assay with Motavizumab. Day 56 sera of mice immunized with RSVF, FFLM or NRM was diluted 1:100 and SPR response units (RU) were measured on sensor chip surfaces containing the respective immunogen. Motavizumab binding sites were then blocked by saturating amounts of Motavizumab, and the residual serum response was measured to calculate the serum fraction competed by Motavizumab binding. Mice immunized with FFLM or NRM show significantly higher (**** p < 0.0001, Mann-Whitney U test) levels of serum antibodies that are competed by Motavizumab binding. **d)** Site II specific serum titers at day 56 from mice immunized with RSVF, FFLM and NRM, measured by ELISA against site II peptide. Three immunizations with prefusion RSVF elicited low levels of site II specific antibodies, whereas FFLM and NRM vaccinations yielded significantly higher peptide specific serum titers (**** p < 0.0001, Mann-Whitney U test). Data shown are derived from at least two independent experiments, each sample assayed in duplicates.

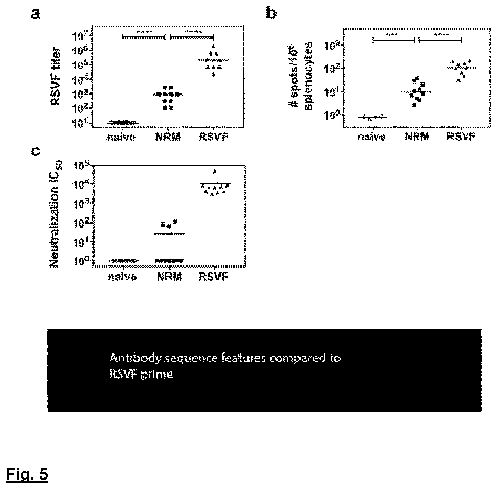

**Fig. 5. RSVF cross-reactivity and serum neutralization. a)** NRM elicits prefusion RSVF cross-reactive antibodies, which are two orders of magnitude lower compared to prefusion RSVF immunization (**** $p < 0.0001$, Mann-Whitney U test). Mice immunized only with adjuvant (naive) do not show RSVF cross-reactivity. **b)** B-cell ELISpot of mouse splenocytes to quantify prefusion RSVF specific antibody secreting cells (ASC). Number of ASCs per $10^6$ splenocytes that secrete prefusion RSVF specific antibodies following three immunizations with adjuvant only (naive), NRM or prefusion RSVF (*** $p = 0.0007$, **** $p < 0.0001$, Mann-Whitney U test). **c)** RSV neutralizing activity of mouse sera from day 56 shown as neutralization $IC_{50}$. Three out of ten mice immunized with NRM showed detectable RSV neutralizing activity, whereas all mice immunized with prefusion RSVF neutralized RSV (mean $IC_{50} = 10,827$). Data shown are from one out of two independent experiments. **d)** Next generation sequencing of antibody repertoire.

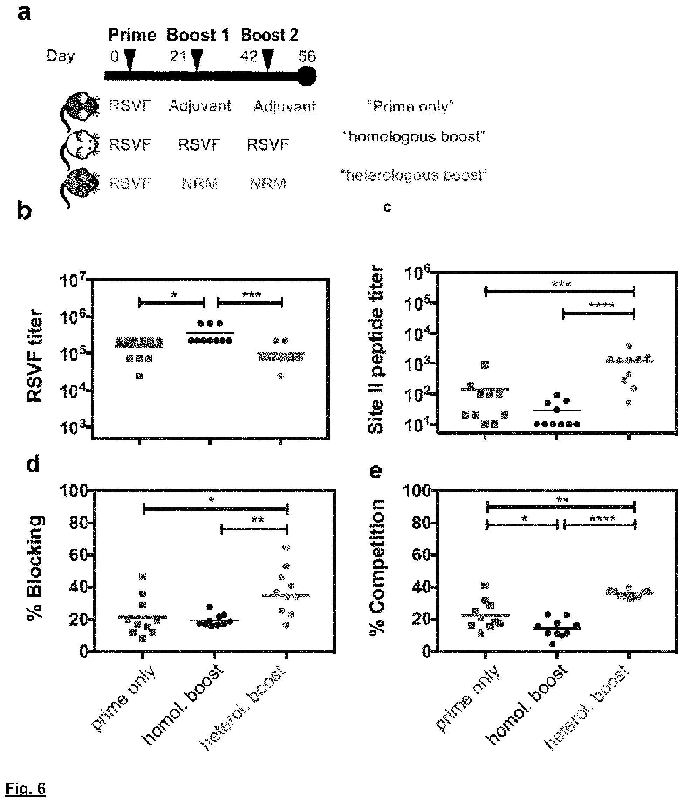

**Fig. 6. Heterologous prime boost reshapes antibody responses enhancing levels of site II specific antibodies. a)** Heterologous prime-boost study groups. Three mouse cohorts were immunized with either 1x RSVF ("prime only"), 3x RSVF ("homologous boost") or 1x RSVF followed by two boosts with NRM ("heterologous boost"), **b)** Antibody titers directed against prefusion RSVF. Mice receiving three immunizations show slightly higher RSVF specific serum titers (* $p = 0.02$, Mann-Whitney U test) compared to the RSVF prime only. Mice receiving a heterologous boost showed statistically comparable titers to the RSVF prime only group, whereas the difference between the homologous and heterologous boost cohort was statistically significant (*** $p = 0.0004$, Mann-Whitney U test). **c)** Site II specific titers measured by ELISA showed that heterologous boost significantly increases site II specific titers compared to both prime and homologous boost groups (*** $p = 0.0009$ and **** $p < 0.0001$, respectively, Mann-Whitney U test). Albeit not statistically significant ($p = 0.06$), mice receiving a homologous boost had lower levels of site II specific antibodies compared to prime only group. **d)** SPR competition assay with Motavizumab on a prefusion RSVF coated sensor chip. Sera from indicated groups were diluted 1:100 and RSVF binding responses were quantified. Site II was then blocked with Motavizumab, and the remaining serum response quantified. The heterologous boost induced a significantly higher fraction of site II directed antibodies competed with Motavizumab for RSVF binding, as compared to both prime only and homologous boost groups (*$p = 0.02$ and ** $p = 0.002$, respectively, Mann-Whitney U test). **e)** Quantification of site II specific responses in a competition ELISA. Binding was measured against prefusion RSVF, and the Area Under the Curve (AUC) was calculated in presence of NRM competitor, normalized to the AUC in presence of RSVN as control competitor. Compared to the prime only group, the homologous boost resulted in significantly lower site II specific serum titers, confirming the trend observed in c) (* $p = 0.03$, Mann-Whitney U test). The heterologous boost increased the fraction of site II targeting antibodies within the pool of prefusion RSVF specific antibodies compared to both control groups (** $p = 0.001$ and **** $p < 0.0001$, Mann-Whitney U test). Data presented are from at least two independent experiments, each sample assayed in duplicates.

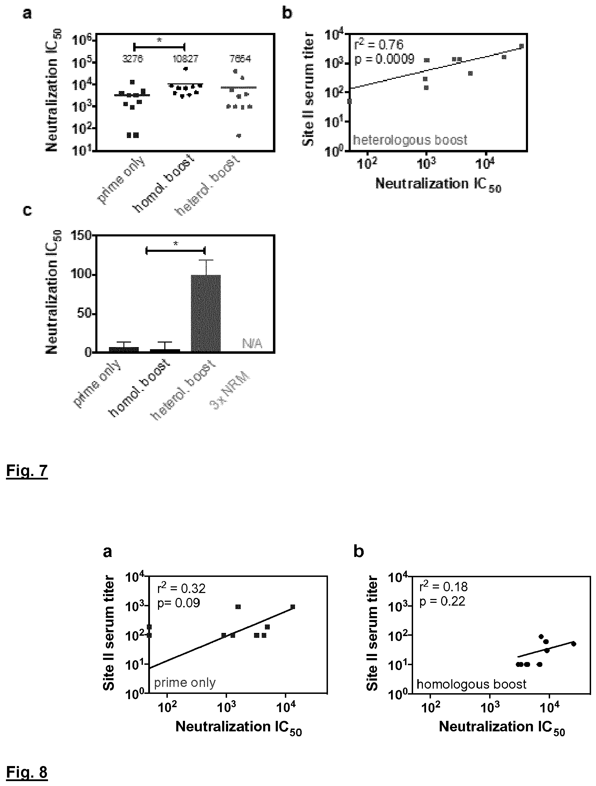

**Fig. 7. Boosted site II specific antibodies are functional and mediate the increased neutralization activity. a)** RSV serum neutralization $IC_{50}$ values are presented for each group. Compared to the prime only group, mice receiving a homologous boost showed increased RSV neutralization titers (* $p = 0.01$, Mann-Whitney U test). On average, the heterologous boost yielded 2-fold increased serum neutralization titers compared to prime only, but these differences were statistically non-significant both compared to prime only as well as to the homologous boost group. **b)** Correlation of site II specific serum titer (measured by peptide ELISA) with RSV neutralization $IC_{50}$ as determined for each mouse within the heterologous prime boost cohort. Correlations for control groups are shown in Figure 8. Data represent the mean of two independent experiments, each measured in duplicates. Pearson correlation coefficient ($r^2$) and p-value were calculated in GraphPad Prism. **c)** Site II specific antibody fractionation reveals increased levels of neutralizing antibodies. Site II specific antibodies from mouse sera were enriched in an affinity purification. Site II specific antibodies isolated from heterologous boost group showed 15-fold increased RSV neutralizing activity compared to both control groups (* $p = 0.02$, Mann-Whitney U test). No site II mediated neutralization was detected for mice receiving three immunizations of NRM. Data are presented from two independent experiments, and each sample was assayed in duplicates with additional controls shown in Figure 9.

**Fig. 8. Correlation of site II specific serum titer (measured by peptide ELISA) with RSV neutralization IC50.** Correlation for mouse cohort receiving only a prefusion RSVF priming immunization (a) and the homologous boost cohort (b). Data represent the mean of two independent experiments, each measured in duplicates. Pearson correlation coefficients (r2) and p-values were calculated in GraphPad Prism.

**Fig. 9. Serum fractionation and enrichment of site II specific antibodies.** a) Experimental scheme. Streptavidin agarose beads were conjugated to biotinylated antigenic site II peptide. As control, unconjugated streptavidin beads were prepared. Sera from ten mice within each cohort was pooled and mixed with conjugated and unconjugated beads. Column flow-through was analyzed by ELISA (b), and eluted site II specific antibodies were analyzed in an RSV neutralization assay (c). b) ELISA against antigenic site II peptide of column flow-through. Immunization groups as described in Figure 6 (prime only, homologous boost and heterologous boost). ELISA signal (OD at 450 nm) is shown for column flow-through from serum fractionation shown in (a). Streptavidin beads that were not coupled to antigenic site II were used as controls, and did not deplete site II reactivity in the flow through. Data and error bars presented are averaged from two different experiments. c) Example RSV neutralization assay curves from elution fraction, using site II conjugated (black) or unconjugated streptavidin beads (grey). Luciferase signal is plotted on the y-axis, and is a measure for RSV replication as previously reported (M. A. Rameix-Welti et al., Visualizing the replication of respiratory syncytial virus in cells and in living mice. Nat Commun 5, 5104 (2014)). The elution fraction is diluted as indicated on the x-axis. Data shown are from one experiment performed in duplicates.

**Fig. 10. Amino acid sequences.** The amino acid sequence of FFLM is shown as SEQ ID NO: 1. Underlined residues in SEQ ID NO: 1 are those which are conserved between FFLM and FFL_001. The amino acid sequence of the carrier, RSVN derived from human RSV strain Long, ATCC VR-26, is shown as SEQ ID NO: 2. The full length fusion protein of FFLM-RSVN is shown as SEQ ID NO: 3. The two sequences (FFLM and RSVN) are joined by a linker, here the tripeptide linker GSW (shown in bold). Antigenic site II peptide sequence is shown as SEQ ID NO: 4.

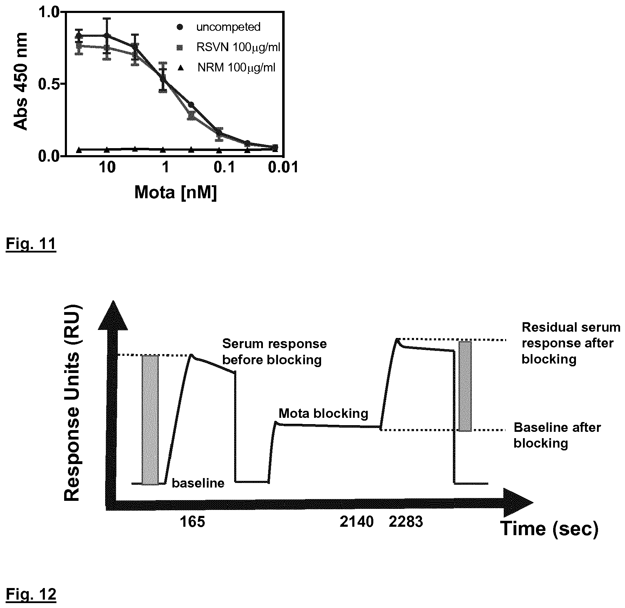

**Fig. 11. Competition ELISA with Motavizumab antibody control.** Plates were coated with prefusion RSVF as described in methods. A 3-fold serial dilution of Motavizumab (starting at 30 nM) was prepared in presence of different competitors, either the unconjugated RSV nucleoprotein nanoparticle (RSVN), the epitope scaffold nanoparticle (NRM), or none. Following overnight competition, binding of Motavizumab to RSVF was measured. RSVN competition did not affect RSVF binding of Motavizumab as expected. In contrast, NRM efficiently competed with RSVF for Motavizumab binding at the indicated competitor concentration. Data shown are from one experiment, with error bars derived from technical duplicates.

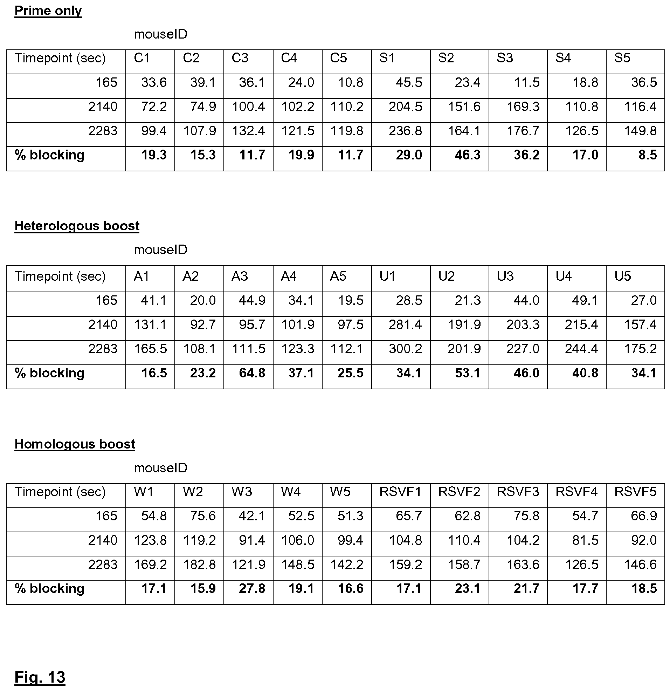

**Fig. 12. Schematic representation of the Surface Plasmon Resonance competition assay.** Mouse sera is injected on an antigen coated sensor chip surface to measure initial response (orange). Following regeneration, Motavizumab binding sites are blocked by excess of Motavizumab. Residual serum response is determined on a blocked surface (blue). For data analysis, response units at indicated timepoints are extracted, and % competition is calculated as described in methods and shown in the table of Fig 13.

**Fig. 13. Response units from SPR competition assay for prime boost immunization regimen.** Timepoints as shown in Fig. 12.

DETAILED DESCRIPTION OF THE INVENTION

Here, we investigate whether a computationally designed immunogen presenting a single epitope is able to reshape serum antibody responses, under conditions of preexisting immunity, towards the increased recognition of a specific neutralizing epitope. For these studies, we used an optimized formulation and resurfaced version of the previously designed RSV site II epitope-scaffold which is targeted by the monoclonal antibody Palivizumab (*21*), the only FDA approved prophylactic measure against RSV infection.

We show that an optimized, multivalent presentation of an RSV antigenic site II scaffold elicits superior levels of epitope-specific antibodies compared to prefusion RSVF in naive mice, indicating that the immunodominance of a particular epitope can be altered through its presentation in a distinct molecular context. We further used the site II scaffold as a boosting immunogen in animals primed with prefusion RSVF, mimicking a scenario of preexisting immunity against RSVF. Whereas homologous boosting immunizations with prefusion RSVF failed to increase site II specific antibodies, we found that heterologous boosts with an epitope scaffold nanoparticle enhanced serum responses towards this *bona fide* vaccine target, and that the boosted antibodies neutralized RSV *in vitro.* Overall, we provide for the first-time compelling evidence that synthetic immunogens with a single epitope can efficiently enhance subdominant neutralizing antibody responses *in vivo* and redirect antibody specificities in bulk antibody responses. Such strategy may present a valid alternative for pathogens for which future vaccines are required to reshape pre-existing immunity and elicit finely tuned antibody specificities.

Design of an RSV based nanoparticle displaying a site II epitope-focused immunogen

In a previous study, an RSV site II epitope-scaffold nanoparticle was shown to elicit serum neutralization activity in NHPs (*21*). Despite the fact that very potent monoclonal antibodies were isolated from the immunized NHPs, the neutralization potency at the serum level was modest. Therefore, our first aim was to take the best previously tested immunogen (FFL_001) and to further optimize the immunogen delivery and immunization conditions to maximize the induction of site II specific antibodies. To test the effect of different adjuvants with the single site epitope-scaffold FFL_001, we immunized four groups of mice with FFL_001 in combination with four different, commonly used, adjuvants (Adjuplex™, polyIC, AddaVax™ and Alumhydrogel). As assessed by serum ELISA, FFL_001 adsorbed to Alumhydrogel showed highest overall immunogenicity according to the antibody titers against the immunogen and elicited antibodies cross-reactive with prefusion RSVF in four out of five mice (Fig. 1).

Next, we sought to develop an improved, easily producible nanoparticle to multimerize the epitope-scaffold for efficient B-cell receptor crosslinking. Previously, Correia et al. (21) employed a chemical conjugation strategy of FFL_001 to an Hepatitis-B core antigen (HbcAg) based nanoparticle, which inevitably resulted in a hard to handle construct with a laborious purification process. In recent studies, Riffault and colleagues (39) have reported the use of RSV nucleoprotein (RSVN) as a nanoparticle platform for immunogen presentation. We reasoned that RSVN would be an ideal particle platform to multimerize an RSV epitope scaffold, as RSVN contains strong, RSV directed T cell epitopes (40). However, our initial attempts to genetically fuse FFL_001 to RSVN yielded insoluble proteins. We therefore employed structure-based protein resurfacing (41), attempting to improve the solubility of this site II epitope-scaffold when arrayed in high density on RSVN. To guide our resurfacing design process, we leveraged information from a sequence homolog of the ribosomal recycling factor (PDB: 1ISE), the structural template originally used to design FFL_001. Based on a sequence alignment of the mouse homolog (NCBI reference: NP_080698.1) and FFL_001, we exchanged the FFL_001 amino acids for the mouse sequence homolog and used Rosetta Fixed Backbone Design (42) to ensure that the mutations where not energetically unfavorable, resulting in 38 amino acid substitutions (34.2% overall). We named this variant FFLM, and the yields of expression for this variant in *E. coli* showed a five-fold increase when compared to FFL_001, and was confirmed to be monomeric in solution (Fig. 2).

To confirm that the resurfacing did not alter the epitope integrity, we measured the binding affinities using Surface Plasmon Resonance (SPR) of FFLM to Motavizumab and a panel of human site II nAbs previously isolated (6). All antibodies bound with high affinity to FFLM, indicating a broad reactivity of this immunogen with a diverse panel of human neutralizing antibodies (Figure 3). Interestingly, the tested nAbs showed about one order of magnitude higher affinity of the epitope-scaffold as compared to prefusion RSVF, suggesting that the epitope is properly presented and likely further stabilized in a relevant conformation. Importantly, the FFLM-RSVN fusion protein expressed with high yields in E. coli (>10 mg/liter), and formed a nanoring particle, dubbed NRM, that was monodisperse in solution with approximately 21 nm of diameter (Figure 3). Although we cannot fully rationalize the factors that contributed to the solubility improvement upon multimerization, our strategy to transplant surface residues from a sequence homolog to synthetic proteins may prove useful to enhance the solubility of other computationally designed proteins.

NRM enhances the induction of site II specific antibodies

We next tested the immunogenicity of the site II scaffold nanoring and its ability to elicit site II specific antibodies. Three groups of ten mice were subjected to three immunizations with 10 μg of NRM, monomeric FFLM and prefusion RSVF (43) which is currently the leading immunogen for an RSV vaccine (Figure 4a). Based on the results of our adjuvant screen (Fig. 1), all the immunogens were formulated in Alumhydrogel, an adjuvant approved for human use. As expected, NRM showed a higher overall immunogenicity than monomeric FFLM (Figure 4b).

A key aspect of epitope-focused based vaccines is to understand how much of the antibody response targets the viral epitope presented to the immune system. Therefore, we sought to measure the site II specific antibody titers elicited by NRM and FFLM, and compare these epitope specific antibody responses to those elicited by prefusion RSVF. Using an SPR competition assay to measure site II specific antibodies in sera (described in methods), we observed that NRM elicited superior site II specific antibody responses than RSVF (Figure 4c). This was surprising given that the ratio of site II epitope surface area to overall immunogen surface is similar in NRM and RSVF (Fig. 2). To confirm this finding through a direct binding assay rather than a competitive format, we measured the binding levels of sera to the site II epitope in a peptide ELISA, where the site II peptide was immobilized on streptavidin coated wells. Consistent with the previous experiment, we found that NRM elicited 100-fold higher site II specific responses than RSVF (Figure 4d). Together, these data validated NRM as our lead immunogen to induce high levels of site II specific antibodies in mice.

NRM induces low levels of RSVF cross-reactive antibodies with low neutralization potency

Given the substantial site II specific serum titers elicited by NRM in mice, we investigated whether these antibodies cross-reacted with prefusion RSVF and were sufficient to neutralize RSV *in vitro*.

Following three immunizations with NRM, all the mice (N=10) developed detectable serum cross-reactivity with prefusion RSVF (mean serum titer = 980) (Figure 5a). Unsurprisingly, the overall quantity of prefusion RSVF cross-reactive antibodies upon immunization with an immunogen presenting a single epitope is more than two orders of magnitude lower than those of mice immunized with prefusion-RSVF. Similarly, a B-cell ELISpot revealed that NRM immunized animals presented prefusion RSVF reactive antibody secreting cells, but their frequency was approximately one order of magnitude lower than upon immunization with prefusion RSVF (Figure 5).

The major determinant for antibody specificity is attributed the heavy chain CDR3 region (HCDR3) (44). While for certain classes of neutralizing antibodies the antibody lineages and their sequence features are well defined (e.g. HIV neutralizing VRC01 class antibodies (45), or RSV neutralizing MPE8-like antibodies (46)), antibodies targeting RSV antigenic site II seem to derive from diverse precursors and do not show HCDR3 sequence convergence in humans (6). While we did not expect to find dominant specific lineages or HCDR3 sequence patterns in mice, we used next generation sequencing to ask whether NRM could elicit antibodies with similar sequence signatures to those elicited by prefusion RSVF. Indeed, we found eleven clonotypes, defined as antibodies deriving from the same VH gene with the same HCDR3 length and 80% sequence similarity, that overlapped between NRM and the prefusion RSVF immunized cohort, suggesting that at the molecular level relevant antibody lineages can be activated with the NRM immunogen.

We further investigated whether these low levels of prefusion RSVF binding antibodies were sufficient to accomplish RSV neutralization *in vitro*. While three immunizations with prefusion RSVF elicited potent RSV neutralizing serum titers (mean $IC_{50}$= 10,827), for NRM we only detected low levels of RSV neutralizing serum activity in three out of ten mice. This result is consistent with Correia *et al.* (21), who observed no serum neutralization in mice, but succeeded to induce neutralizing antibodies in prior RSV seronegative NHPs.

Altogether, we concluded that despite the NRM's superior potential to induce high levels of site II specific antibodies, the majority of antibodies activated from the naive repertoire is unable to potently neutralize RSV *in vitro*. A likely explanation stemming from the structural comparison between the epitope-focused immunogen (FFLM) and RSVF, is that although epitope specific antibodies are abundantly elicited by NRM, these antibodies do not recognize the site II epitope in its native quaternary environment of RSVF on the prefusion conformation and on virions with sufficient amounts and affinity to potently neutralize RSV.

NRM boosts site II specific antibodies under conditions of preexisting immunity

While RSV vaccination studies in naive animal models are an important first step to validate novel immunogens, the most important human target populations for RSV vaccination are pregnant women and the elderly. These two target groups are seropositive for RSVF due to life-long recurrent infections and have a basal level of RSV nAbs (47). We hypothesized that the high affinity of the epitope scaffold towards a panel of site II specific nAbs, together with the ability to elicit high titers of site II specific antibodies *in vivo*, could make the NRM an efficient immunogen to recall RSV primed site II specific B-cells and thereby achieving an enhanced and focused neutralization response.

Our initial homologous prime-boost immunizations studies with prefusion RSVF showed that site II specific responses were subdominant (Figure 4C and 4D). Given that this is a common immunological phenotype for many of the neutralization epitopes that are relevant for vaccine development (48), we sought to test if NRM could boost subdominant site II specific antibody lineages that should ultimately be functional and recognize the epitope in the tertiary environment of prefusion RSVF. To test this hypothesis, we designed a mouse immunization experiment with three cohorts as outlined in Figure 6a. Following a priming immunization with RSVF, cohort (1) was boosted with adjuvant only ("prime only"), cohort (2) received two boosting immunization with prefusion RSVF ("homologous boost"), and cohort (3) received two boosts with NRM ("heterologous boost").

A comparison between prefusion RSVF immunized groups prime only and homologous boost revealed that the two additional boosting immunizations with RSVF only slightly increased overall titers of prefusion RSVF specific antibodies (p=0.02, Mann-Whitney), indicating that a single immunization with adjuvanted RSVF is sufficient to induce close-to-maximal serum titers against RSVF (Figure 6b). Following the heterologous boost with NRM, overall RSVF specific antibody titers remained statistically comparable to the prime only group (p=0.22, Mann-Whitney).

Next, we quantified the site II specific endpoint serum titers in a peptide ELISA format (Figure 6c). Interestingly, the homologous boost with prefusion RSVF failed to increase site II specific antibody levels, reducing the responses directed to site II to our lower limit of detection by ELISA. This result was yet another example of the underlying complexity inherent to the fine specificity of antibody responses elicited by complex immunogens and how important specificities can be dampened throughout the development of an antibody response. In contrast to the homologous boost, the heterologous boost with NRM significantly increased epitope specific serum titers (p<0.0001, Mann-Whitney).

In order to understand whether this increase relied at least partially on an actual recall of antibodies primed by RSVF, or rather an independent antibody response that is irrelevant for RSVF binding and RSV neutralization, we dissected the epitope specificity within the RSVF-specific serum response.

To confirm that the peptide ELISA results were robust, we first performed an SPR competition assay to quantify antibody levels that compete with Motavizumab for prefusion RSVF binding. A significantly higher fraction (p=0.02, Mann-Whitney) of prefusion RSVF reactive antibodies were competed by Motavizumab in mouse sera primed with prefusion RSVF and boosted with NRM (mean % competition = 37.5±14.5%), as compared to mice immunized once or three times with prefusion RSVF (21.5% ± 12.1% or 19.5% ± 3.7%, respectively) (Figure 6d). Similarly, a competition ELISA revealed that a significantly larger fraction of the overall RSVF reactivity was directed to site II specific antibodies upon heterologous boost, as compared to both control groups (36.1% $\pm$ 2.5% versus 22.6% $\pm$ 9.1% or 14.4% $\pm$5.9%, respectively, p=0.002 and p<0.0001, Mann-Whitney). In contrast, site II specific antibodies were significantly lower when comparing mice that received only one RSVF prime to mice receiving three RSVF immunizations, indicating that RSVF boosting immunizations depleted site II specific antibody titers (p=0.03, Mann-Whitney) (Figure 6e). Together, we have shown that the serum antibody specificity can be steered towards antigenic site II by boosting with NRM under conditions of preexisting immunity, and thereby increase site II specific antibody levels *in vivo*. This is an important and distinctive feature of NRM compared to prefusion RSVF, which was shown to decrease Palivizumab-like antibody responses under the same conditions. These results might have broad implications on strategies to control antibody fine specificities in vaccination schemes, both for RSV and other pathogens.

Boosted antibodies neutralize RSV *in vitro*

The enhanced reactivity to site II observed in the heterologous prime-boost scheme led us to investigate if changing the nature of the immune response had functional consequences on RSV neutralization. Overall, we observed 2.3-fold higher serum neutralization titers in mice receiving a heterologous boost (mean $IC_{50}$=7654) compared to the prime only control group (mean $IC_{50}$=3275), as shown in Figure 7a. While the neutralization titers difference in these two groups (prime vs. heterologous boost) did not reached statistical significance, the homologous prime boost was significantly improved relative to the prime only (mean $IC_{50}$ = 10,827 vs mean $IC_{50}$ = 3,276) and interestingly we also did not observe a statistically significance between the homologous boost and the heterologous boost cohort (mean $IC_{50}$ = 10,827 vs. mean $IC_{50}$ = 7,654).

Next, we assessed if this increase in neutralization was driven by increased levels of Palivizumab-like antibodies. We observed that site II directed antibody levels correlated with overall serum neutralization titers in the heterologous prime boost group ($r^2$=0.76, p=0.0009) (Figure 7b), whereas prime only ($r^2$=0.32, p=0.09) or animals receiving a homologous boost showed no such correlation ($r^2$ = 0.18, p=0.22) (Figure 8). To characterize the neutralizing serum activity dependent on antigenic site II, we pooled mouse sera within each cohort, enriched site II specific antibodies and measured neutralization (see methods). Briefly, we incubated pooled sera from each group with streptavidin beads conjugated to biotin-labeled antigenic site II peptide, and eluted bound antibodies. To control for the quality of the enrichment protocol we verified by ELISA that the column flow-through was depleted of site II specific antibodies (Figure 9).

Strikingly, mice receiving a heterologous boost showed a 15-fold increased site II mediated neutralization as compared to mice immunized once or three times with prefusion RSVF (Figure 7c). We performed the same experiment for mice immunized three times with NRM and did not detect any RSV neutralization in this format. This observation is consistent with the very low levels of bulk sera neutralization measured in this group, indicating that NRM can only boost neutralizing antibodies under conditions of preexisting immunity. Together, we conclude that the heterologous boosting scheme with a single epitope immunogen enhanced subdominant neutralizing antibody responses directed to the antigenic site presented, and effectively redirected an antibody-based immune response *in vivo*.

**Discussion**

Despite a rapid increase of our atomic-level understanding of antibody-antigen interactions for various pathogens, the translation of structural information into efficacious immunogens eliciting an antibody response specific to such *bona fide* remains a key challenge for next generation vaccine development. In recent years, numerous nAbs have been identified targeting different epitopes on pre- and postfusion RSVF, and particularly prefusion specific nAbs have been proposed for further clinical development and provided a wealth of valuable vaccine design targets (49). Despite these recent advances, the proven clinical success of Palivizumab (50) supports the induction of high levels of Palivizumab-like antibodies as a key route of vaccine development for both naive and RSV seropositive individuals.

Epitope scaffolds have been shown to hold great potential to elicit RSV antigenic site II specific, neutralizing antibody responses in naive non-human primates, and an elicited monoclonal antibody showed superior neutralization potency as compared to Palivizumab (21). However, a major limitation of epitope scaffolds or peptide based vaccine approaches (51-53) is that the quaternary environment of the epitope in a viral protein is lost, and thereby the binding mode of a significant fraction of the antibodies elicited is likely incompatible with the epitope in its native environment. This is reinforced by our finding that albeit NRM elicited high serum levels of site II directed antibodies, only residual RSV neutralizing activity was observed, which is consistent with several previous studies using epitope scaffolds (53-55). This sets a major limitation for the use of synthetic scaffolds in an epitope focused vaccine approach in naive individuals.

However, relevant target populations to receive an RSV vaccine (pregnant women and elderly), have preexisting humoral responses against RSVF, including to site II (56). To date, boosting epitope specific, neutralizing antibodies under conditions of preexisting immunity has proven challenging, particularly because a strong antibody response against immunodominant epitopes that structurally surround a subdominant neutralization epitope can sterically mask the neutralization epitope, preventing a subdominant epitope from mounting a potent antibody response (27, 29,

*30, 57*). Several studies have addressed the difficulty of overcoming immunodominance under preexisting immunity conditions, which seem to be impacted by multiple factors such as serological antibody levels, their specificity, memory B-cell counts, adjuvants as well as the immunization or infection route (*28*). Heterologous prime boost schemes are a promising strategy to guide the fine specificity of antibody responses and to focus antibody responses on antigenic sites of vulnerability. Several vaccine studies have been conducted for pathogens as Influenza (*37, 38*), RSV (*35*), and HIV (*32*), where the heterologous immunogens were modified versions of the viral fusion proteins, and therefore, not heterologous to the same degree as a computationally designed, single epitope scaffold. An important point to consider related to immunogens based on modified versions of viral proteins is if in fact immunodominant signatures do not remain, and thereby may steer away the antibody responses from the target epitopes. While this scenario may not be fully absent in the synthetic epitope-scaffolds, it is at least mitigated by the fact that the protein has not evolved under the evolutionary pressure of escaping the immune system.

Another key aspect in heterologous prime-boost schemes is to maintain the structural integrity of the neutralizing epitope in both priming and heterologous boosting immunogen. FFL_001 has been thoroughly structurally characterized, and presents a very close mimicry of the site II epitope in RSVF. We hypothesized that the strong binding affinity of FFLM for a diverse panel of nAbs would allow FFLM to recall site II specific, memory B-cells, and thereby increase site II specific serum titers *in vivo*. Given the very low serum titers specific for antigenic site II following a prefusion RSVF prime, it was questionable whether NRM could boost these subdominant antibody levels. However, previous studies with HIV immunogens have shown that a very low number of epitope specific B-cells (1 out of 1,000,000) is sufficient to be selected and recruited to the germinal center by a multimeric immunogen that binds with high affinity to the target B-cell receptor (*54*). Indeed, we found that under conditions where site II specific antibodies are subdominant, a heterologous boost can redirect serum antibody specificity towards increased site II recognition. Importantly, these antibodies competed with Motavizumab for RSVF binding, confirming their recognition of the epitope in its native quaternary context. In contrast, repeated immunization with prefusion RSVF failed to direct antibodies towards antigenic site II.

This study demonstrates that the number of neutralization epitopes in a heterologous boosting immunogen can be reduced to a single, well-defined site, which, if the immunogen is optimally presented, can enhance preexisting, subdominant antibody responses targeting this epitope. The ability to focus antibody responses narrowly to a single, well understood epitope that, once targeted by sufficient titers of antibodies, mediates clinical protection, is an important milestone for RSV vaccine development.

Given that this approach is generalizable and epitope scaffold nanoparticles prove successful in boosting antibodies specific for other sites, this strategy holds great potential to tune levels of antibody specificities depending on the target population. Goodwin et al. have shown that young infants preferentially develop RSV site III specific antibodies that lack somatic hypermutation (*46*). Consequently, vaccine antigens given to pregnant women should primarily induce antibodies targeting distal antigenic sites, to avoid interference with an immune response mounted by the infant. Given these specific antibody lineage requirements and the previously failed clinical trial in infants, its seems unlikely that a prefusion RSVF based vaccine will cover all the target populations, but could rather require precision immunogens to spotlight different antigenic sites dependent on the target population. An increasing structural and immunological understanding of antibody specificities will help to inform the design of novel immunogens as well as appropriate immunization regimens to tailor vaccine induced antibody responses to different target populations.

Our results, however, show that the success of this boosting strategy is dependent on the presence of a low, but detectable amount of site II specific antibodies that were elicited by prefusion RSVF. Although three immunizations with NRM yielded even higher site II peptide specific antibodies, these antibodies were largely non-neutralizing. Further studies in more relevant animal models will reveal if natural infection with RSV can also serve as appropriate prime for subsequent boosting of site II neutralizing antibodies, as well as for other RSV neutralization epitopes.

Projecting beyond the RSV vaccine problem, these results provide a strong rationale for developing precision immunogens that present conserved, neutralization sensitive sites of other pathogens. For example, multiple epitopes on influenza hemagglutinin head and stem region that confer broad and potent antibody mediated neutralization have been identified, and focusing antibody responses on these defined epitopes may remove the need for annual vaccine reformulation, and may also protect against emerging pandemic strains (*14, 48, 58, 59*). The Influenza vaccine problem seems particularly well suited for our approach, considering that the human population has pre-existing immunity to influenza which may include some subdominant bnAbs that seasonal vaccines fail to stimulate. Additionally, antigenically related viruses such as Zika and Dengue pose a major challenge for vaccine development, as antibodies mounted against the envelope protein of Dengue can facilitate infection with Zika (*60*). A site conserved between the Dengue and Zika envelope protein has been structurally characterized and suggested for the development of an epitope focused immunogen (*8*).

A challenge when seeking to apply the immunofocusing strategy to other antigenic sites and pathogens is the development of epitope scaffolds that can stably present the epitope in a synthetic immunogen that is compatible with antibody binding. While the RSV antigenic site II is structurally a simple helix-turn-helix motif, many other neutralization epitopes identified are comprised of multiple, discontinuous segments. However, continuous advances in rational protein design techniques (*61*) will allow to design more complex protein scaffolds to stabilize increasingly complex epitopes.

Altogether, we have shown the development of an optimized RSV epitope scaffold nanoparticle able to boost subdominant, neutralizing antibody responses *in vivo* that target site II, an epitope of high clinical relevance. This is a distinctive feature compared to prefusion RSVF as boosting immunogen, and underlines how subdominant epitopes can be converted to immunodominant epitopes when presented in a different environment. We foresee great potential of this strategy to overcome the challenge of boosting and focusing preexisting immunity towards defined neutralization epitopes, potentially applicable for multiple pathogens.

**Methods**

Resurfacing

The previously published RSV site II epitope scaffold ("FFL_001") (21) was designed based on a crystal structure of a mutant of ribosome recycling factor from E. coli (PDB entry 1ISE. Using BLAST, we identified sequence homologs of 1ISE from eukaryotic organisms, and create a multiple sequence alignment with clustal omega (CLUSTALO (1.2.1)) (62) of the mouse homolog sequence (NCBI reference NP_080698.1), 1ISE and FFL_001. Surface exposed residues of FFL_001 were then mutated to the respective residue of the mouse homolog using the Rosetta fixed backbone design application (42), resulting in 38 surface mutations. Amino acid changes were verified to not impact overall Rosetta energy score term.

Protein expression and purification

**FFLM**

DNA sequences of the epitope-scaffold designs were purchased from Genscript and cloned in pET29b, in frame with a C-terminal 6x His tag. Plasmid was transformed in *E. coli* BL21 (DE3), and grown in Terrific Broth supplemented with Kanamycin (50 $\mu$g/ml). Cultures were inoculated to an $OD_{600}$ of 0.1 from an overnight culture and incubated at 37°C. After reaching $OD_{600}$ of 0.6, expression was induced by the addition of 1 mM isopropyl-$\beta$-D-thiogalactoside (IPTG) and cells were incubated for further 4-5h at 37°C. Cell pellets were resuspended in lysis buffer (50 mM TRIS, pH 7.5, 500 mM NaCl, 5% Glycerol, 1 mg/ml lysozyme, 1 mM PMSF, 1 $\mu$g/ml DNase) and sonicated on ice for a total of 12 minutes, in intervals of 15 seconds sonication followed by a 45 seconds pause. Lysates were clarified by centrifugation (18,000 rpm, 20 minutes), sterile-filtered and purified using a His-Trap FF column on an Äkta pure system (GE healthcare). Bound proteins were eluted in buffer containing 50 mM Tris, 500 mM NaCl and 300 mM imidazole, pH 7.5. Concentrated proteins were further purified by size exclusion chromatography on a Superdex™ 75 300/10 (GE Healthcare) in PBS. Protein concentrations were determined via measuring the absorbance at 280 nm on a Nanodrop (Thermo Scientific). Proteins were concentrated by centrifugation (Millipore, #UFC900324) to 1 mg/ml, snap frozen in liquid nitrogen and stored at -80°C.

**NRM**

The full-length N gene (sequence derived from the human RSV strain Long, ATCC VR-26; GenBank accession number AY911262.1) was PCR amplified using the Phusion DNA polymerase (Thermo Scientific) and cloned into pET28a+ at Ncol-Xhol sites to obtain the pET-N plasmid. The sequence of FFLM was then PCR amplified and cloned into pET-N at Ncol site to the pET-NRM plasmid. *E. coli* BL21 (DE3) bacteria were co-transformed with pGEX-PCT (*63*) and pET-FFLM-N plasmids and grown in LB medium containing ampicillin (100 $\mu$g/ml) and kanamycin (50 $\mu$g/ml). The same volume of LB medium was then added, and protein expression was induced by the addition of 80 $\mu$g/ml (IPTG) to the medium. Bacteria were incubated for 15 h at 28°C and then harvested by centrifugation. For protein purification, bacterial pellets were resuspended in lysis buffer (50 mM Tris-HCl [pH 7.8], 60 mM NaCl, 1 mM EDTA, 2 mM dithiothreitol [DTT], 0.2% Triton X-100, 1 mg/ml lysozyme) supplemented with a complete protease inhibitor cocktail (Roche, Mannheim, Germany), incubated for one hour on ice, and disrupted by sonication. The soluble fraction was collected by centrifugation at 4°C for 30 min at 10,000 x g. Glutathione-Sepharose 4B beads (GE Healthcare, Uppsala, Sweden) were added to clarify supernatants and incubated at 4°C for 15h. The beads were then washed two times in 20 mM Tris pH 8.5, 150 mM NaCl. Purified recombinant NRM proteins were loaded onto a Sephacryl S-200 HR 16/30 column (GE Healthcare) and eluted in 20 mM Tris-HCl pH 8.5; 150 mM NaCl.

**Antibody variable fragments (Fabs)**

For Fab expression, heavy and light chain DNA sequences were purchased from Twist Biosciences and cloned separately into pHLSec mammalian expression vector (Addgene, #99845) using AgeI and XhoI restriction sites. Expression plasmids were pre-mixed in a 1:1 stoichiometric ratio and co-transfected into HEK293-F cells and cultured in Freestyle™ medium (Gibco, #12338018). Supernatants were harvested after one week by centrifugation and purified using a kappa-select column (GE Healthcare). Elution of bound proteins was conducted using a 0.1 M glycine buffer (pH 2.7) and eluates were immediately neutralized by the addition of 1 M TRIS ethylamine (pH 9), followed by buffer exchange to PBS pH 7.4.

**Respiratory Syncytial Virus Fusion protein (prefusion RSVF)**

Protein sequence of prefusion RSVF corresponds to the sc9-10 DS-Cav1 A149C Y458C S46G E92D S215P K465Q variant designed by Joyce et al., 2016 (*43*), to which we refer to as RSVF DS2. RSVF DS2 was codon optimized for mammalian expression, and cloned into pHCMV-1 vector, together with two c-terminal Strep-Tag II and one 8x His tag. Plasmids were transfected in HEK293-F cells and cultured in FreeStyleTM medium. Supernatants were harvested one week after transfection, and purified via Ni-NTA affinity chromatography. Bound protein was eluted using a buffer containing 10mM Tris, 500mM NaCl and 300mM Imidazole (pH 7.5), and eluate was further purified on a StrepTrap HP affinity column (GE Healthcare, #29-0486-53). Bound protein was eluted in 10mM Tris, 150mM NaCl and 20mM Desthiobiotin (Sigma, #D-1411), pH8, and size excluded in PBS, pH 7.4, on a Superdex 200 Increase 10/300 GL column (GE healthcare) to obtain trimeric RSVF.

Affinity determination using Surface Plasmon Resonance

Surface Plasmon Resonance experiments were performed on a Biacore 8K at room temperature with HBS-EP+ running buffer (10 mM HEPES pH 7.4, 150 mM NaCl, 3mM EDTA, 0.005% v/v Surfactant P20) (GE Healthcare). Approximately 100 response units of FFLM were immobilized via amine coupling on a CM5 sensor chip (GE Healthcare). Serial dilutions of site II specific antibody variable fragments (Fabs) were injected as analyte at a flow rate of 30 $\mu$l/min with 120 seconds contact time. Following each injection cycle, ligand regeneration was performed using 0.1 M glycine at pH 2. Data analysis was performed using 1:1 Langmuir binding kinetic fits within the Biacore evaluation software (GE Healthcare).

Mouse immunizations

All animal experiments were approved by the Veterinary Authority of the Canton of Vaud (Switzerland) according to Swiss regulations of animal welfare (animal protocol number 3074). Six-week-old, female Balb/c mice were ordered from Janvier labs and acclimatized for one week. Immunogens were thawed on ice, and diluted in PBS pH 7.4 to a concentration of 0.2 mg/ml. The immunogens were then mixed with an equal volume of 2% Alumhydrogel (Invivogen, # vac-alu-250), resulting in a final Alumhydrogel concentration of 1%. Other adjuvants were formulated according to manufacturer's instructions. After mixing for one hour at 4°C, each mouse was injected with 100 $\mu$l, corresponding to 10 $\mu$g immunogen adsorbed to Alumhydrogel. All immunizations were done subcutaneously, with no visible irritation around the injection site. Immunizations were performed on day 0, 21 and 42. 100-200 $\mu$l blood were drawn on day 0, 14, 35 and the maximum amount of blood (200-1000$\mu$l) was taken by cardiac puncture at day 56, when mice were sacrificed.

Antigen ELISA

Nunc Medisorp plates (Thermo Scientific, # 467320) were coated overnight at 4°C with 100 $\mu$l of antigen (recombinant pusion RSVF, FFLM and NRM) diluted in coating buffer (100 mM sodium bicarbonate, pH 9) at a final concentration of 0.5 $\mu$g/ml. For blocking, plates were incubated for two hours at room temperature with blocking buffer (PBS + 0.05% Tween 20 (PBST) supplemented with 5% skim milk powder (Sigma, #70166)). Mouse sera were serially diluted in blocking buffer, and incubated for one hour at room temperature. Plates were washed five times with PBST, before adding 100 $\mu$l of anti-mouse HRP conjugated secondary antibody diluted in blocking buffer (abcam, #ab99617, dilution 1:1500). Additional five wash steps were performed before adding Pierce TMB substrate (Thermo Scientific, # 34021). Reaction was stopped by adding 100 $\mu$l of 2M sulfuric acid, and absorbance at 450 nm was measured on a Tecan Safire 2 plate reader.

Each plate contained a standard curve of Motavizumab to normalize signals of different plates and experiments. Normalization was done in GraphPad Prism. The mean value was plotted for each cohort and statistical analysis was performed using GraphPad Prism.

Competition ELISA

Prior to incubation with coated antigen plate, sera were serially diluted in presence of 100 μg/ml competitor antigen, and incubated overnight at 4°C. Curves were plotted using GraphPad Prism, and the area under the curve (AUC) was calculated for the specific (NRM) and control (RSVN) competitor. % competition was calculated using the following formula (64):

$$\% \text{ competition} = (1 - (\frac{AUC(specific\ competitor\ (NRM))}{AUC(control\ competitor\ (NR))})) * 100$$

Peptide sandwich ELISA

The antigenic site II was synthesized as peptide by JPT Peptide Technologies, Germany. The following sequence was synthesized, and biotinylated at the N-terminus:

MLTNSELLSKINDMPITNDQKKLMSNNVQI (SEQ ID NO: 4)

For ELISA analysis of peptide-reactive serum antibodies, Nunc MediSorp plates were coated with 5 μg/ml streptavidin (Thermo Scientific, #21122) for one hour at 37°C. Subsequently, ELISA plates were blocked as indicated above, followed by the addition of 2.4 μg/ml of the biotinylated site II peptide. Coupling was performed for one hour at room temperature. The following steps were performed as described for the antigen ELISA.

Serum competition using Surface Plasmon Resonance

Approximately 300 response units (RU) of antigen were immobilized through amine coupling. Mouse sera were diluted 1:100 in running buffer and flowed over the prepared CM5 chip with a contact of 120 secs to obtain an initial response unit ($RU_{\text{non-blocked surface}}$). The surface was regenerated using 50 mM NaOH. Sequentially, Motavizumab was injected four times at a concentration of 2 μM leading to complete blocking of Motavizumab binding sites as confirmed by signal saturation. The same serum dilution was reinjected to determine the remaining response ($RU_{\text{blocked surface}}$). The delta serum response ($\Delta SR$) corresponds to the baseline subtracted, maximum signal of the injected sera.

$$\Delta SR = RU_{\text{(non-)blocked surface}} - RU_{\text{Baseline}}$$

Percent blocking was calculated as follows:

$$\% \text{ blocking} = (1 - (\frac{\Delta SR_{\text{blocked surface}}}{\Delta SR_{\text{non-blocked surface}}})) * 100$$

Enzyme-linked immunospot assay (ELISPOT)

B-cell ELISPOT assays were performed using the Mouse IgG ELISpot HRP kit (Mabtech, #3825-2H) according to the manufacturer's instructions. Briefly, mouse spleens were isolated, and pressed through a cell strainer (Corning, #352350) to obtain a single cell suspension. Splenocytes were resuspended in RPMI media (Gibco, #11875093) supplemented with 10% FBS, Penicillin/Streptomycin, 0.01 μg/ml IL2, 1 μg/ml R848 and 50 μM β-mercaptoethanol for -60 hours stimulation at 37 °C, 5% $CO_2$. ELISpot plates (PVDF 96-well plates, Millipore, #MSIPS4510) were coated overnight with 15 μg/ml antigen diluted in PBS, followed by careful washing and blocking using RPMI + 10% FCS. Live splenocytes were counted and cell number adjusted to $1\times10^7$ cells/ml. Serial dilutions of splenocytes were plated in duplicates, and incubated overnight with coated plates. After several wash steps with PBS buffer, plates were incubated for two hours with biotinylated anti-mouse total IgG (Mabtech, #3825-6-250) in PBS, followed by incubation with streptavidin conjugated to horseradish peroxidase (HRP, Mabtech, #3310-9) for one hour. Spots were revealed using tetramethylbenzidine (TMB, Mabtech, #3651-10), and counted with an automatic reader (Bioreader 2000; BioSys GmbH). Results were represented as number of spots per $10^6$ splenocytes.

RSV neutralization assay

The RSV A2 strain carrying a luciferase gene (RSV-Luc) was a kind gift of Marie-Anne Rameix-Welti, UFR des Sciences et de la Santé, Paris. HepG2 cells were seeded in Corning 96-well tissue culture plates (Sigma, #CLS3595) at a density of 40,000 cells/well in 100 μl of Minimum Essential Medium (MEM, Gibco, #11095-080) supplemented with 10% FBS (Gibco, 10500-084), L-glutamine 2 mM (Gibco, #25030-081) and Penicillin-Streptomycin (Gibco, #15140-122), and grown overnight at 37 °C with 5% CO2.

Sera was heat inactivated for 30 minutes at 56 °C. Serial, two-fold dilutions were prepared in an untreated 96 well plate using MEM without phenol red (M0, Life Technologies, #51200-038) + 2mM L-glutamine + 100 IU/mL Penicillin + 100 μg/mL Streptomycin, and mixed with 800 pfu/well RSV-Luc (corresponding to a final MOI of 0.01). After incubating diluted sera and virus for one hour at 37 °C, growth media was removed from HepG2 cell layer and 100 μl/well of the serum-virus mixture added. After 48 hours, cells were lysed in 100 μl buffer containing 32 mM Tris pH 7.9, 10 mM MgCl$_2$, 1.25% Triton X-100, 18.75% glycerol and 1mM DTT. 50 μl lysate were transferred to a 96-well plate with white background (Sigma, # CLS3912). 50 μl of lysis buffer supplemented with 1 μg/ml luciferin (Sigma, #L-6882) and 2 mM ATP (Sigma, #A3377) were added to each well immediately before reading luminescence signal on a Tecan Infinite 500 plate reader.

On each plate, a Palivizumab dilution series was included to ensure comparability of neutralization data. In our assay, we determined IC$_{50}$ values for Palivizumab of 0.32 μg/ml, which is similar to what other groups have determined (65). The neutralization curve was plotted and fitted using the GraphPad variable slope fitting model, weighted by $1/Y^2$.

Sera fractionation

400 μl of streptavidin agarose beads (Thermo Scientific, #20347) were pelleted at 13,000 rpm for 2 minutes in a table top centrifuge, and washed with Phosphate buffered saline (PBS). 200 μg of biotinylated site II peptide were incubated for 2 hours at room temperature to allow coupling of biotinylated peptide to streptavidin beads. Beads were washed three times with 1 ml of PBS to remove excess of peptide, and resuspended to a total volume of 500 μl bead slurry. Mouse sera from the same cohort (N=10) were pooled (4 μl each, 40 μl total) in a total volume of 200 μl PBS, and 90 μl diluted sera were mixed with 150 μl of bead slurry, followed by an overnight incubation at 4 °C. Beads were pelleted by centrifugation, and the supernatant carefully removed by pipetting. Beads were then washed twice with 200 μl PBS, and the wash fractions discarded. To elute site II specific antibodies, beads were resuspended in 200 μl elution buffer (0.1 M glycine, pH 2.7) and incubated for 1 minute before centrifugation. Supernatant was removed, neutralized with 40 μl neutralization buffer (1 M Tris pH 7.5, 300 mM NaCl), and stored at -20 °C for subsequent testing for RSV neutralization. As control, unconjugated streptavidin was used for each sample to account for non-specific binding.

**References**

1. R. Rappuoli, C. W. Mandl, S. Black, E. De Gregorio, Vaccines for the twenty-first century society. Nat Rev Immunol 11, 865-872 (2011).

2. S. A. Plotkin, Correlates of protection induced by vaccination. Clin Vaccine Immunol 17, 1055-1065 (2010).

3. F. Sesterhenn, J. Bonet, B. E. Correia, Structure-based immunogen design-leading the way to the new age of precision vaccines. Curr Opin Struct Biol 51, 163-169 (2018).

4. D. R. Burton, L. Hangartner, Broadly Neutralizing Antibodies to HIV and Their Role in Vaccine Design. Annu Rev Immunol 34, 635-659 (2016).

5. P. S. Lee, I. A. Wilson, Structural characterization of viral epitopes recognized by broadly cross-reactive antibodies. Curr Top Microbiol Immunol 386, 323-341 (2015).

6. M. S. Gilman et a/., Rapid profiling of RSV antibody repertoires from the memory B cells of naturally infected adult donors. Sci Immunol 1, (2016).

7. J. S. McLellan, Neutralizing epitopes on the respiratory syncytial virus fusion glycoprotein. Curr Opin Virol 11, 70-75 (2015).

8. G. Barba-Spaeth et al., Structural basis of potent Zika-dengue virus antibody cross-neutralization. Nature 536, 48-53 (2016).

9. D. F. Robbiani et a/., Recurrent Potent Human Neutralizing Antibodies to Zika Virus in Brazil and Mexico. Cell 169, 597-609 e511 (2017).

10. M. Xu et a/., A potent neutralizing antibody with therapeutic potential against all four serotypes of dengue virus. NPJ Vaccines 2, 2 (2017).

11. Z. A. Bornholdt et a/., Isolation of potent neutralizing antibodies from a survivor of the 2014 Ebola virus outbreak. Science 351, 1078-1083 (2016).

12. S. Chandramouli et al., Structural basis for potent antibody-mediated neutralization of human cytomegalovirus. Sci Immunol 2, (2017).

13. K. M. Hastie et al., Structural basis for antibody-mediated neutralization of Lassa virus. Science 356, 923-928 (2017).

14. D. Corti et al., A neutralizing antibody selected from plasma cells that binds to group 1 and group 2 influenza A hemagglutinins. Science 333, 850-856 (2011).

15. J. S. McLellan et al., Structure of RSV fusion glycoprotein trimer bound to a prefusion-specific neutralizing antibody. Science 340, 1113-1117 (2013).

16. D. R. Burton, Antibodies, viruses and vaccines. Nat Rev Immunol 2, 706-713 (2002).

17. D. R. Burton, What Are the Most Powerful Immunogen Design Vaccine Strategies? Reverse Vaccinology 2.0 Shows Great Promise. Cold Spring Harb Perspect Biol 9, (2017).

18. J. S. McLellan et al., Structure-based design of a fusion glycoprotein vaccine for respiratory syncytial virus. Science 342, 592-598 (2013).

19. A. Impagliazzo et al., A stable trimeric influenza hemagglutinin stem as a broadly protective immunogen. Science 349, 1301-1306 (2015).

20. H. M. Yassine et al., Hemagglutinin-stem nanoparticles generate heterosubtypic influenza protection. Nat Med 21, 1065-1070 (2015).

21. B. E. Correia et al., Proof of principle for epitope-focused vaccine design. Nature 507, 201-206 (2014).

22. M. F. Delgado et al., Lack of antibody affinity maturation due to poor Toll-like receptor stimulation leads to enhanced respiratory syncytial virus disease. Nat Med 15, 34-41 (2009).

23. A. M. Killikelly, M. Kanekiyo, B. S. Graham, Pre-fusion F is absent on the surface of formalin-inactivated respiratory syncytial virus. Sci Rep 6, 34108 (2016).

24. I. Widjaja et al., Characterization of Epitope-Specific Anti-Respiratory Syncytial Virus (Anti-RSV) Antibody Responses after Natural Infection and after Vaccination with Formalin-Inactivated RSV. J Virol 90, 5965-5977 (2016).

25. R. T. Stein et al., Respiratory syncytial virus hospitalization and mortality: Systematic review and meta-analysis. Pediatr Pulmonol 52, 556-569 (2017).

26. B. B. Finlay, G. McFadden, Anti-immunology: evasion of the host immune system by bacterial and viral pathogens. Cell 124, 767-782 (2006).

27. S. F. Andrews et al., Immune history profoundly affects broadly protective B cell responses to influenza. Sci Transl Med 7, 316ra192 (2015).

28. D. Angeletti et al., Defining B cell immunodominance to viruses. Nat Immunol 18, 456-463 (2017).

29. V. I. Zarnitsyna et al., Masking of antigenic epitopes by antibodies shapes the humoral immune response to influenza. Philos Trans R Soc Lond B Biol Sci 370, (2015).

30. V. I. Zarnitsyna, J. Lavine, A. Ellebedy, R. Ahmed, R. Antia, Multi-epitope Models Explain How Pre-existing Antibodies Affect the Generation of Broadly Protective Responses to Influenza. PLoS Pathog 12, el005692 (2016).

31. G. D. Victora, M. C. Nussenzweig, Germinal centers. Annu Rev Immunol 30, 429-457 (2012).

32. B. Briney et al., Tailored Immunogens Direct Affinity Maturation toward HIV Neutralizing Antibodies. Cell 166, 1459-1470 e1411 (2016).

33. J. G. Jardine et al., HIV-1 VACCINES. Priming a broadly neutralizing antibody response to HIV-1 using a germline-targeting immunogen. Science 349, 156-161 (2015).

34. M. Silva et al., Targeted Elimination of Immunodominant B Cells Drives the Germinal Center Reaction toward Subdominant Epitopes. Cell Rep 21, 3672-3680 (2017).

35. J. C. Boyington et al., Structure-Based Design of Head-Only Fusion Glycoprotein Immunogens for Respiratory Syncytial Virus. PLoS One 11, e0159709 (2016).

36. G. A. Kirchenbaum, D. M. Carter, T. M. Ross, Sequential Infection in Ferrets with Antigenically Distinct Seasonal H1N1 Influenza Viruses Boosts Hemagglutinin Stalk-Specific Antibodies. J Virol 90, 1116-1128 (2016).

37. K. Van Reeth et al., Heterologous prime-boost vaccination with H3N2 influenza viruses of swine favors cross-clade antibody responses and protection. NPJ Vaccines 2, (2017).

38. C. J. Wei et al., Induction of broadly neutralizing H1N1 influenza antibodies by vaccination. Science 329, 1060-1064 (2010).

39. P. L. Herve et al., RSV N-nanorings fused to palivizumab-targeted neutralizing epitope as a nanoparticle RSV vaccine. Nanomedicine 13, 411-420 (2017).

40. R. G. Tawar et al., Crystal structure of a nucleocapsid-like nucleoprotein-RNA complex of respiratory syncytial virus. Science 326, 1279-1283 (2009).

41. B. E. Correia et al., Computational protein design using flexible backbone remodeling and resurfacing: case studies in structure-based antigen design. J Mol Biol 405, 284-297 (2011).

42. B. Kuhlman, D. Baker, Native protein sequences are close to optimal for their structures. Proc Natl Acad Sci U S A 97, 10383-10388 (2000).

43. M. G. Joyce et al., Iterative structure-based improvement of a fusion-glycoprotein vaccine against RSV. Nat Struct Mol Biol 23, 811-820 (2016).

44. J. L. Xu, M. M. Davis, Diversity in the CDR3 region of V(H) is sufficient for most antibody specificities. Immunity 13, 37-45 (2000).

45. J. F. Scheid et al., Sequence and structural convergence of broad and potent HIV antibodies that mimic CD4 binding. Science 333, 1633-1637 (2011).

46. E. Goodwin et al., Infants Infected with Respiratory Syncytial Virus Generate Potent Neutralizing Antibodies that Lack Somatic Hypermutation. Immunity 48, 339-349 e335 (2018).

47. L. J. Anderson et al., Strategic priorities for respiratory syncytial virus (RSV) vaccine development. Vaccine 31 Suppl 2, B209-215 (2013).

48. N. L. Kallewaard et al., Structure and Function Analysis of an Antibody Recognizing All Influenza A Subtypes. Cell 166, 596-608 (2016).

49. I. Rossey, J. S. McLellan, X. Saelens, B. Schepens, Clinical Potential of Prefusion RSV F-specific Antibodies. Trends Microbiol 26, 209-219 (2018).

50. A. Mejias, O. Ramilo, Review of palivizumab in the prophylaxis of respiratory syncytial virus (RSV) in high-risk infants. Biologics 2, 433-439 (2008).

51. G. Ofek et al., Elicitation of structure-specific antibodies by epitope scaffolds. Proc Natl Acad Sci U S A 107, 17880-17887 (2010).

52. N. Jaberolansar et al., Induction of high titred, non-neutralising antibodies by self-adjuvanting peptide epitopes derived from the respiratory syncytial virus fusion protein. Sci Rep 7, 11130 (2017).

53. J. S. McLellan et al., Design and characterization of epitope-scaffold immunogens that present the motavizumab epitope from respiratory syncytial virus. J Mol Biol 409, 853-866 (2011).

54. R. K. Abbott et al., Precursor Frequency and Affinity Determine B Cell Competitive Fitness in Germinal Centers, Tested with Germline-Targeting HIV Vaccine Immunogens. Immunity 48, 133-146 e136 (2018).

55. B. E. Correia et al., Computational design of epitope-scaffolds allows induction of antibodies specific for a poorly immunogenic HIV vaccine epitope. Structure 18, 1116-1126 (2010).

56. J. O. Ngwuta et al., Prefusion F-specific antibodies determine the magnitude of RSV neutralizing activity in human sera. Sci Transl Med 7, 309ra162 (2015).

57. S. F. Andrews et al., High preexisting serological antibody levels correlate with diversification of the influenza vaccine response. J Virol 89, 3308-3317 (2015).

58. D. C. Ekiert et al., A highly conserved neutralizing epitope on group 2 influenza A viruses. Science 333, 843-850 (2011).

59. D. D. Raymond et al., Conserved epitope on influenza-virus hemagglutinin head defined by a vaccine-induced antibody. Proc Natl Acad Sci U S A 115, 168-173 (2018).

60. W. Dejnirattisai et al., Dengue virus sero-cross-reactivity drives antibody-dependent enhancement of infection with zika virus. Nat Immunol 17, 1102-1108 (2016).

61. A. Chevalier et al., Massively parallel de novo protein design for targeted therapeutics. Nature 550, 74-79 (2017).

62. F. Sievers et al., Fast, scalable generation of high-quality protein multiple sequence alignments using Clustal Omega. Mol Syst Biol 7, 539 (2011).

63. N. Castagne et al., Biochemical characterization of the respiratory syncytial virus P-P and P-N protein complexes and localization of the P protein oligomerization domain. J Gen Virol 85, 1643-1653 (2004).

64. D. W. Kulp et al., Structure-based design of native-like HIV-1 envelope trimers to silence non-neutralizing epitopes and eliminate CD4 binding. Nat Commun 8, 1655 (2017).

65. H. Wu et al., Development of motavizumab, an ultra-potent antibody for the prevention of respiratory syncytial virus infection in the upper and lower respiratory tract. J Mol Biol 368, 652-665 (2007).

**Claims**

1. A polypeptide comprising or consisting of an amino acid sequence according to SEQ ID NO: 1, or comprising or consisting of an amino acid sequence which is 95% or more identical to that of SEQ ID NO: 1.

2. A polypeptide according to claim 1, which further comprises a carrier polypeptide sequence.

3. A polypeptide according to claim 2, wherein the carrier polypeptide comprises or consists of a) an RSVN protein sequence; or b) the amino acid sequence of SEQ ID NO: 2; or c) an amino acid sequence which is 95% or more identical to that of SEQ ID NO: 2.

**4.** A nucleic acid sequence encoding a polypeptide according to any of claims 1 to 3.

**5.** A nanoparticle comprising multiple copies of a polypeptide according to any of claims 1 to 3.

**6.** A polypeptide according to any of claims 1 to 3, or a nucleic acid sequence according to claim 4, or a nanoparticle according to claim 5, for use as a vaccine.

**7.** A vaccine composition comprising a polypeptide according to any of claims 1 to 3, or a nucleic acid sequence according to claim 4, or a nanoparticle according to claim 5, optionally comprising one or more pharmaceutically acceptable carriers, and/or adjuvants.

**8.** A vaccine composition according to claim 7, wherein the adjuvant is alhydrogel.

**9.** The vaccine composition of claim 7 or 8, wherein the vaccine is for administration to subjects, preferably human subjects, who have been previously exposed to RSV.

**10.** The vaccine composition of any of claims 7 to 9, wherein the composition is for administration subsequent to an initial priming vaccination with one or more RSV-derived proteins or glycoproteins, preferably RSVF.

**11.** The vaccine composition of any of claims 7 to 10 wherein the composition further comprises one or more additional RSV-derived proteins or glycoproteins, preferably RSVF.

**12.** A kit of parts comprising a vaccine composition according to any of claims 7 to 11, and a further vaccine composition comprising an RSV-derived protein or glycoprotein, preferably RSVF.

**13.** A polypeptide according to any of claims 1 to 3, or a nucleic acid sequence according to claim 4, or a nanoparticle according to claim 5, for use in a method for immunising a subject against RSV, the method comprising

a) administering a vaccine composition comprising said polypeptide, nucleic acid, or nanoparticle to a subject; and
b) prior to said administration step, administering a further vaccine composition comprising an RSV-derived protein or glycoprotein, preferably the RSVF glycoprotein; or wherein the vaccine composition(s) are administered to a subject who has previously been exposed to RSV infection.

**14.** The polypeptide, nucleic acid, or nanoparticle of claim 13 wherein two or more administrations of the composition of step (a) are administered.

**15.** A combination comprising a) a first priming vaccine comprising an immunogen obtained or obtainable from a selected pathogen, wherein the immunogen provides a desired subdominant immune response and a dominant immune response; and b) a second boost vaccine comprising a synthetic immunogen, said synthetic immunogen comprising a polypeptide sequence designed to mimic a desired epitope recognised by said subdominant immune response, said polypeptide sequence being fused to a carrier polypeptide sequence; for use in a method for boosting subdominant immune responses to said selected pathogen.

**Fig 1**

**a**

CLUSTAL O(1.2.4) multiple sequence alignment

```
FFL_001    GSRSDMRKDAERRFDKFVEAAKNKFDKFKAALRKGDIKEERRKDMKKLARKEAEQARRAV    60
FFLM       ASREDMREEADEDFKSFVEAAKDNFNKFKARLRKGKITREHREMMKKLAKQNANKAKEAV    60
           .**.***::*:. *..******:*:**** ****.*..*:*: *****::::*::*:.**

FFL_001    RNRLSELLSKINDMPITNDQKKLMSNDVLKFAAEAEKKIEALAADAEDKFTQ 112
FFLM       RKRLSELLSKINDMPITNDQKKLMSNQVLQFADDAEAEIDQLAADATKEFTG 112
           *:************************:**:** :** :*: ***** .:**
```

**b**

**c**

Comparison of antigenic site II surface area to overall immunogen surface area

**Fig 2**

Fig. 3

Fig. 4

EP 3 628 678 A1

**Fig. 5**

21

Fig. 6

**a**

**b**

**c**

**Fig. 7**

**a**

**b**

**Fig. 8**

## Fig. 9

>FFLM (SEQ ID NO: 1)

ASREDMREEADEDFKSFVEAAKDNFNKFKARLRKGKITREHREMMKKLAKQNANKAKEAVRKRLSELLSKINDM
PITNDQKKLMSNQVLQFADDAEAEIDQLAADATKEFTG

>RSVN (SEQ ID NO: 2)

GSGSMALSKVKLNDTLNKDQLLSSSKYTIQRSTGDSIDTPNYDVQKHINKLCGMLLITEDANHKFTGLIGMLYAMS
RLGREDTIKILRDAGYHVKANGVDVTTHRQDINGKEMKFEVLTLASLTTEIQINIEIESRKSYKKMLKEMGEVAPEY
RHDSPDCGMIILCIAALVITKLAAGDRSGLTAVIRRANNVLKNEMKRYKGLLPKDIANSFYEVFEKHPHFIDVFVHFG
IAQSSTRGGSRVEGIFAGLFMNAYGAGQVMLRWGVLAKSVKNIMLGHASVQAEMEQVVEVYEYAQKLGGEAGF
YHILNNPKASLLSLTQFPHFSSVVLGNAAGLGIMGEYRGTPRNQDLYDAAKAYAEQLKENGVINYSVLDLTAEELE
AIKHQLNPKDNDVEL

>NRM (SEQ ID NO: 3)

ASREDMREEADEDFKSFVEAAKDNFNKFKARLRKGKITREHREMMKKLAKQNANKAKEAVRKRLSELLSKINDM
PITNDQKKLMSNQVLQFADDAEAEIDQLAADATKEFTGGSWGSGSMALSKVKLNDTLNKDQLLSSSKYTIQRSTG
DSIDTPNYDVQKHINKLCGMLLITEDANHKFTGLIGMLYAMSRLGREDTIKILRDAGYHVKANGVDVTTHRQDING
KEMKFEVLTLASLTTEIQINIEIESRKSYKKMLKEMGEVAPEYRHDSPDCGMIILCIAALVITKLAAGDRSGLTAVIRR
ANNVLKNEMKRYKGLLPKDIANSFYEVFEKHPHFIDVFVHFGIAQSSTRGGSRVEGIFAGLFMNAYGAGQVMLRW
GVLAKSVKNIMLGHASVQAEMEQVVEVYEYAQKLGGEAGFYHILNNPKASLLSLTQFPHFSSVVLGNAAGLGIMG
EYRGTPRNQDLYDAAKAYAEQLKENGVINYSVLDLTAEELEAIKHQLNPKDNDVEL

>antigenic site II (SEQ ID NO: 4)

MLTNSELLSKINDMPITNDQKKLMSNNVQI

## Fig. 10

Fig. 11

Fig. 12

**Prime only**

mouseID

| Timepoint (sec) | C1 | C2 | C3 | C4 | C5 | S1 | S2 | S3 | S4 | S5 |
|---|---|---|---|---|---|---|---|---|---|---|
| 165 | 33.6 | 39.1 | 36.1 | 24.0 | 10.8 | 45.5 | 23.4 | 11.5 | 18.8 | 36.5 |
| 2140 | 72.2 | 74.9 | 100.4 | 102.2 | 110.2 | 204.5 | 151.6 | 169.3 | 110.8 | 116.4 |
| 2283 | 99.4 | 107.9 | 132.4 | 121.5 | 119.8 | 236.8 | 164.1 | 176.7 | 126.5 | 149.8 |
| **% blocking** | **19.3** | **15.3** | **11.7** | **19.9** | **11.7** | **29.0** | **46.3** | **36.2** | **17.0** | **8.5** |

**Heterologous boost**

mouseID

| Timepoint (sec) | A1 | A2 | A3 | A4 | A5 | U1 | U2 | U3 | U4 | U5 |
|---|---|---|---|---|---|---|---|---|---|---|
| 165 | 41.1 | 20.0 | 44.9 | 34.1 | 19.5 | 28.5 | 21.3 | 44.0 | 49.1 | 27.0 |
| 2140 | 131.1 | 92.7 | 95.7 | 101.9 | 97.5 | 281.4 | 191.9 | 203.3 | 215.4 | 157.4 |
| 2283 | 165.5 | 108.1 | 111.5 | 123.3 | 112.1 | 300.2 | 201.9 | 227.0 | 244.4 | 175.2 |
| **% blocking** | **16.5** | **23.2** | **64.8** | **37.1** | **25.5** | **34.1** | **53.1** | **46.0** | **40.8** | **34.1** |

**Homologous boost**

mouseID

| Timepoint (sec) | W1 | W2 | W3 | W4 | W5 | RSVF1 | RSVF2 | RSVF3 | RSVF4 | RSVF5 |
|---|---|---|---|---|---|---|---|---|---|---|
| 165 | 54.8 | 75.6 | 42.1 | 52.5 | 51.3 | 65.7 | 62.8 | 75.8 | 54.7 | 66.9 |
| 2140 | 123.8 | 119.2 | 91.4 | 106.0 | 99.4 | 104.8 | 110.4 | 104.2 | 81.5 | 92.0 |
| 2283 | 169.2 | 182.8 | 121.9 | 148.5 | 142.2 | 159.2 | 158.7 | 163.6 | 126.5 | 146.6 |
| **% blocking** | **17.1** | **15.9** | **27.8** | **19.1** | **16.6** | **17.1** | **23.1** | **21.7** | **17.7** | **18.5** |

**Fig. 13**

Europäisches
Patentamt

European
Patent Office

Office européen
des brevets

## EUROPEAN SEARCH REPORT

Application Number

EP 18 19 7259

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRUNO E. CORREIA ET AL: "Proof of principle for epitope-focused vaccine design", NATURE, vol. 507, no. 7491, 5 February 2014 (2014-02-05), pages 201-206, XP055528790, London ISSN: 0028-0836, DOI: 10.1038/nature12966 * abstract * * page 202, left-hand column, paragraph 2 * * page 202, right-hand column, last paragraph - page 203, right-hand column, paragraph 1 * * page 204, right-hand column, last paragraph - page 205, right-hand column, paragraph 1 * * figure 3 * ----- | 1-15 | INV. C07K14/135 C12N7/00 |
| X | US 2015/050306 A1 (SCHIEF WILLIAM R [US] ET AL) 19 February 2015 (2015-02-19) * paragraphs [0033], [0126] - [0130] * ----- -/-- | 1-15 | TECHNICAL FIELDS SEARCHED (IPC) C07K C12N |

The present search report has been drawn up for all claims

| Place of search | Date of completion of the search | Examiner |
|---|---|---|
| Munich | 6 December 2018 | Surdej, Patrick |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 3

**Europäisches Patentamt**
**European Patent Office**
**Office européen des brevets**

## EUROPEAN SEARCH REPORT

Application Number

EP 18 19 7259

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PIERRE-LOUIS HERVE ET AL: "RSV N-nanorings fused to palivizumab-targeted neutralizing epitope as a nanoparticle RSV vaccine", NANOMEDICINE: NANOTECHNOLOGY, BIOLOGY AND MEDICINE, vol. 13, no. 2, 20 August 2016 (2016-08-20), pages 411-420, XP055355393, NL ISSN: 1549-9634, DOI: 10.1016/j.nano.2016.08.006 * abstract * * page 411, right-hand column, last paragraph - page 412, left-hand column, paragraph 1 * * page 412, left-hand column, last paragraph - right-hand column, paragraph 1 * * page 413, right-hand column, last paragraph - page 414, left-hand column, paragraph 1 * * page 416, left-hand column, last paragraph - page 417, left-hand column, paragraph 2 * * page 417, left-hand column, paragraph 3 * * page 419, right-hand column, paragraph 3 * * figures 1-2, 6 * ----- -/-- | 1-15 | |

|  |  |  | TECHNICAL FIELDS SEARCHED (IPC) |

The present search report has been drawn up for all claims

| Place of search | Date of completion of the search | Examiner |
|---|---|---|
| Munich | 6 December 2018 | Surdej, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 18 19 7259

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FABIAN SESTERHENN ET AL: "Structure-based immunogen design - leading the way to the new age of precision vaccines", CURRENT OPINION IN STRUCTURAL BIOLOGY, vol. 51, 1 August 2018 (2018-08-01), pages 163-169, XP055528776, GB ISSN: 0959-440X, DOI: 10.1016/j.sbi.2018.06.002 * abstract * * page 163, left-hand column, last paragraph - right-hand column, paragraph 4 * * page 167, left-hand column, paragraph 2 - right-hand column, paragraph 2 * | 1-15 | |
| A | BARNEY S GRAHAM: "Vaccine development for respiratory syncytial virus", CURRENT OPINION IN VIROLOGY, vol. 23, 1 April 2017 (2017-04-01), pages 107-112, XP055528781, United Kingdom ISSN: 1879-6257, DOI: 10.1016/j.coviro.2017.03.012 * the whole document * | 1-15 | TECHNICAL FIELDS SEARCHED (IPC) |

The present search report has been drawn up for all claims

| Place of search | Date of completion of the search | Examiner |
|---|---|---|
| Munich | 6 December 2018 | Surdej, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 7259

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2018

| Patent document cited in search report | | Publication date | Patent family member(s) | | Publication date |
|---|---|---|---|---|---|
| US 2015050306 | A1 | 19-02-2015 | EP | 2834263 A1 | 11-02-2015 |
| | | | US | 2015050306 A1 | 19-02-2015 |
| | | | WO | 2013152274 A1 | 10-10-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006117456 A
- WO 2007119011 A

### Non-patent literature cited in the description

- **CORREIA et al.** reference 21. *Nature,* 2014, vol. 507, 201-206
- **M. A. RAMEIX-WELTI et al.** Visualizing the replication of respiratory syncytial virus in cells and in living mice. *Nat Commun,* 2014, vol. 5 (5104
- **R. RAPPUOLI ; C. W. MANDL ; S. BLACK ; E. DE GREGORIO.** Vaccines for the twenty-first century society. *Nat Rev Immunol,* 2011, vol. 11, 865-872
- **S. A. PLOTKIN.** Correlates of protection induced by vaccination. *Clin Vaccine Immunol,* 2010, vol. 17, 1055-1065
- **F. SESTERHENN ; J. BONET ; B. E. CORREIA.** Structure-based immunogen design-leading the way to the new age of precision vaccines. *Curr Opin Struct Biol,* 2018, vol. 51, 163-169
- **D. R. BURTON ; L. HANGARTNER.** Broadly Neutralizing Antibodies to HIV and Their Role in Vaccine Design. *Annu Rev Immunol,* 2016, vol. 34, 635-659
- **P. S. LEE ; I. A. WILSON.** Structural characterization of viral epitopes recognized by broadly cross-reactive antibodies. *Curr Top Microbiol Immunol,* 2015, vol. 386, 323-341
- **M. S. GILMAN.** Rapid profiling of RSV antibody repertoires from the memory B cells of naturally infected adult donors. *Sci Immunol,* 2016, vol. 1
- **J. S. MCLELLAN.** Neutralizing epitopes on the respiratory syncytial virus fusion glycoprotein. *Curr Opin Virol,* 2015, vol. 11, 70-75
- **G. BARBA-SPAETH et al.** Structural basis of potent Zika-dengue virus antibody cross-neutralization. *Nature,* 2016, vol. 536, 48-53
- **D. F. ROBBIANI.** Recurrent Potent Human Neutralizing Antibodies to Zika Virus in Brazil and Mexico. *Cell,* 2017, vol. 169 (597-609), e511
- **M. XU.** A potent neutralizing antibody with therapeutic potential against all four serotypes of dengue virus. *NPJ Vaccines,* 2017, vol. 2 (2
- **Z. A. BORNHOLDT.** Isolation of potent neutralizing antibodies from a survivor of the 2014 Ebola virus outbreak. *Science,* 2016, vol. 351, 1078-1083
- **S. CHANDRAMOULI et al.** Structural basis for potent antibody-mediated neutralization of human cytomegalovirus. *Sci Immunol,* 2017, vol. 2
- **K. M. HASTIE et al.** Structural basis for antibody-mediated neutralization of Lassa virus. *Science,* 2017, vol. 356, 923-928
- **D. CORTI et al.** A neutralizing antibody selected from plasma cells that binds to group 1 and group 2 influenza A hemagglutinins. *Science,* 2011, vol. 333, 850-856
- **J. S. MCLELLAN et al.** Structure of RSV fusion glycoprotein trimer bound to a prefusion-specific neutralizing antibody. *Science,* 2013, vol. 340, 1113-1117
- **D. R. BURTON.** Antibodies, viruses and vaccines. *Nat Rev Immunol,* 2002, vol. 2, 706-713
- **D. R. BURTON.** What Are the Most Powerful Immunogen Design Vaccine Strategies? Reverse Vaccinology 2.0 Shows Great Promise. *Cold Spring Harb Perspect Biol,* 2017, vol. 9
- **J. S. MCLELLAN et al.** Structure-based design of a fusion glycoprotein vaccine for respiratory syncytial virus. *Science,* 2013, vol. 342, 592-598
- **A. IMPAGLIAZZO et al.** A stable trimeric influenza hemagglutinin stem as a broadly protective immunogen. *Science,* 2015, vol. 349, 1301-1306
- **H. M. YASSINE et al.** Hemagglutinin-stem nanoparticles generate heterosubtypic influenza protection. *Nat Med,* 2015, vol. 21, 1065-1070
- **B. E. CORREIA et al.** Proof of principle for epitope-focused vaccine design. *Nature,* 2014, vol. 507, 201-206
- **M. F. DELGADO et al.** Lack of antibody affinity maturation due to poor Toll-like receptor stimulation leads to enhanced respiratory syncytial virus disease. *Nat Med,* 2009, vol. 15, 34-41
- **A. M. KILLIKELLY ; M. KANEKIYO ; B. S. GRAHAM.** Pre-fusion F is absent on the surface of formalin-inactivated respiratory syncytial virus. *Sci Rep,* 2016, vol. 6, 34108

- **I. WIDJAJA et al.** Characterization of Epitope-Specific Anti-Respiratory Syncytial Virus (Anti-RSV) Antibody Responses after Natural Infection and after Vaccination with Formalin-Inactivated RSV. *J Virol,* 2016, vol. 90, 5965-5977
- **R. T. STEIN et al.** Respiratory syncytial virus hospitalization and mortality: Systematic review and meta-analysis. *Pediatr Pulmonol,* 2017, vol. 52, 556-569
- **B. B. FINLAY ; G. MCFADDEN.** Anti-immunology: evasion of the host immune system by bacterial and viral pathogens. *Cell,* 2006, vol. 124, 767-782
- **S. F. ANDREWS et al.** Immune history profoundly affects broadly protective B cell responses to influenza. *Sci Transl Med,* 2015, vol. 7, 316ra192
- **D. ANGELETTI et al.** Defining B cell immunodominance to viruses. *Nat Immunol,* 2017, vol. 18, 456-463
- **V. I. ZARNITSYNA et al.** Masking of antigenic epitopes by antibodies shapes the humoral immune response to influenza. *Philos Trans R Soc Lond B Biol Sci,* 2015, vol. 370
- **V. I. ZARNITSYNA ; J. LAVINE ; A. ELLEBEDY ; R. AHMED ; R. ANTIA.** Multi-epitope Models Explain How Pre-existing Antibodies Affect the Generation of Broadly Protective Responses to Influenza. *PLoS Pathog,* 2016, vol. 12, el005692
- **G. D. VICTORA ; M. C. NUSSENZWEIG.** Germinal centers. *Annu Rev Immunol,* 2012, vol. 30, 429-457
- **B. BRINEY et al.** Tailored Immunogens Direct Affinity Maturation toward HIV Neutralizing Antibodies. *Cell,* 2016, vol. 166 (1459-1470), e1411
- **J. G. JARDINE et al.** HIV-1 VACCINES. Priming a broadly neutralizing antibody response to HIV-1 using a germline-targeting immunogen. *Science,* 2015, vol. 349, 156-161
- **M. SILVA et al.** Targeted Elimination of Immunodominant B Cells Drives the Germinal Center Reaction toward Subdominant Epitopes. *Cell Rep,* 2017, vol. 21, 3672-3680
- **J. C. BOYINGTON et al.** Structure-Based Design of Head-Only Fusion Glycoprotein Immunogens for Respiratory Syncytial Virus. *PLoS One,* 2016, vol. 11, e0159709
- **G. A. KIRCHENBAUM ; D. M. CARTER ; T. M. ROSS.** Sequential Infection in Ferrets with Antigenically Distinct Seasonal H1N1 Influenza Viruses Boosts Hemagglutinin Stalk-Specific Antibodies. *J Virol,* 2016, vol. 90, 1116-1128
- **K. VAN REETH et al.** Heterologous prime-boost vaccination with H3N2 influenza viruses of swine favors cross-clade antibody responses and protection. *NPJ Vaccines,* 2017, vol. 2
- **C. J. WEI et al.** Induction of broadly neutralizing H1N1 influenza antibodies by vaccination. *Science,* 2010, vol. 329, 1060-1064
- **P. L. HERVE et al.** RSV N-nanorings fused to palivizumab-targeted neutralizing epitope as a nanoparticle RSV vaccine. *Nanomedicine,* 2017, vol. 13, 411-420
- **R. G. TAWAR et al.** Crystal structure of a nucleocapsid-like nucleoprotein-RNA complex of respiratory syncytial virus. *Science,* 2009, vol. 326, 1279-1283
- **B. E. CORREIA et al.** Computational protein design using flexible backbone remodeling and resurfacing: case studies in structure-based antigen design. *J Mol Biol,* 2011, vol. 405, 284-297
- **B. KUHLMAN ; D. BAKER.** Native protein sequences are close to optimal for their structures. *Proc Natl Acad Sci U S A,* 2000, vol. 97, 10383-10388
- **M. G. JOYCE et al.** Iterative structure-based improvement of a fusion-glycoprotein vaccine against RSV. *Nat Struct Mol Biol,* 2016, vol. 23, 811-820
- **J. L. XU ; M. M. DAVIS.** Diversity in the CDR3 region of V(H) is sufficient for most antibody specificities. *Immunity,* 2000, vol. 13, 37-45
- **J. F. SCHEID et al.** Sequence and structural convergence of broad and potent HIV antibodies that mimic CD4 binding. *Science,* 2011, vol. 333, 1633-1637
- **E. GOODWIN et al.** Infants Infected with Respiratory Syncytial Virus Generate Potent Neutralizing Antibodies that Lack Somatic Hypermutation. *Immunity,* 2018, vol. 48 (339-349), e335
- **L. J. ANDERSON et al.** Strategic priorities for respiratory syncytial virus (RSV) vaccine development. *Vaccine,* 2013, vol. 31 (2), B209-215
- **N. L. KALLEWAARD et al.** Structure and Function Analysis of an Antibody Recognizing All Influenza A Subtypes. *Cell,* 2016, vol. 166, 596-608
- **I. ROSSEY ; J. S. MCLELLAN ; X. SAELENS ; B. SCHEPENS.** Clinical Potential of Prefusion RSV F-specific Antibodies. *Trends Microbiol,* 2018, vol. 26, 209-219
- **A. MEJIAS ; O. RAMILO.** Review of palivizumab in the prophylaxis of respiratory syncytial virus (RSV) in high-risk infants. *Biologics,* 2008, vol. 2, 433-439
- **G. OFEK et al.** Elicitation of structure-specific antibodies by epitope scaffolds. *Proc Natl Acad Sci U S A,* 2010, vol. 107, 17880-17887
- **N. JABEROLANSAR et al.** Induction of high titred, non-neutralising antibodies by self-adjuvanting peptide epitopes derived from the respiratory syncytial virus fusion protein. *Sci Rep,* 2017, vol. 7, 11130
- **J. S. MCLELLAN et al.** Design and characterization of epitope-scaffold immunogens that present the motavizumab epitope from respiratory syncytial virus. *J Mol Biol,* 2011, vol. 409, 853-866

- **R. K. ABBOTT et al.** Precursor Frequency and Affinity Determine B Cell Competitive Fitness in Germinal Centers, Tested with Germline-Targeting HIV Vaccine Immunogens. *Immunity,* 2018, vol. 48 (133-146), e136
- **B. E. CORREIA et al.** Computational design of epitope-scaffolds allows induction of antibodies specific for a poorly immunogenic HIV vaccine epitope. *Structure,* 2010, vol. 18, 1116-1126
- **J. O. NGWUTA et al.** Prefusion F-specific antibodies determine the magnitude of RSV neutralizing activity in human sera. *Sci Transl Med,* 2015, vol. 7, 309ra162
- **S. F. ANDREWS et al.** High preexisting serological antibody levels correlate with diversification of the influenza vaccine response. *J Virol,* 2015, vol. 89, 3308-3317
- **D. C. EKIERT et al.** A highly conserved neutralizing epitope on group 2 influenza A viruses. *Science,* 2011, vol. 333, 843-850
- **D. D. RAYMOND et al.** Conserved epitope on influenza-virus hemagglutinin head defined by a vaccine-induced antibody. *Proc Natl Acad Sci U S A,* 2018, vol. 115, 168-173
- **W. DEJNIRATTISAI et al.** Dengue virus sero-cross-reactivity drives antibody-dependent enhancement of infection with zika virus. *Nat Immunol,* 2016, vol. 17, 1102-1108
- **A. CHEVALIER et al.** Massively parallel de novo protein design for targeted therapeutics. *Nature,* 2017, vol. 550, 74-79
- **F. SIEVERS et al.** Fast, scalable generation of high-quality protein multiple sequence alignments using Clustal Omega. *Mol Syst Biol,* 2011, vol. 7, 539
- **N. CASTAGNE et al.** Biochemical characterization of the respiratory syncytial virus P-P and P-N protein complexes and localization of the P protein oligomerization domain. *J Gen Virol,* 2004, vol. 85, 1643-1653
- **D. W. KULP et al.** Structure-based design of native-like HIV-1 envelope trimers to silence non-neutralizing epitopes and eliminate CD4 binding. *Nat Commun,* 2017, vol. 8, 1655
- **H. WU et al.** Development of motavizumab, an ultra-potent antibody for the prevention of respiratory syncytial virus infection in the upper and lower respiratory tract. *J Mol Biol,* 2007, vol. 368, 652-665